# FASCICULE DE BREVET EUROPEEN

(11) **EP 3 947 188 B1**
(45) Date de publication et mention de la délivrance du brevet: **26.07.2023**
(21) Numéro de dépôt: 20723458.4
(22) Date de dépôt: 02.04.2020
(51) Int. Cl.: B65D 77/04, A61F 2/00, B65D 51/20

(54) **EMBALLAGE ET PROCEDE DE DECONDITIONNEMENT CORRESPONDANT**
VERPACKUNG UND ENTSPRECHENDES AUSPACKVERFAHREN
PACKAGE AND CORRESPONDING UNPACKING METHOD

(30) Priorité: 03.04.2019 FR 1903587; 03.04.2019 US 201962828841 P
(43) Date de publication de la demande: 09.02.2022
(73) Titulaire: Selenium Medical, 17000 La Rochelle (FR)
(72) Inventeur: RICHART, Olivier, 17580 Le Bois Plage en Ré (FR)
(74) Mandataire: Ipsilon
(86) Numéro de dépôt international: PCT/FR2020/000080
(87) Numéro de publication internationale: WO 2020/201640

(56) Documents cités:
- WO-A1-2018/078242
- WO-A2-2019/030451
- FR-A1- 2 959 216

## Description

### DOMAINE DE L'INVENTION

La présente invention concerne de manière générale les emballages pour objets, en particulier pour pièces médicales de préférence stérilisées.

### ART ANTERIEUR

On connaît de la demande internationale WO2018078242A1, un emballage comprenant une coque inférieure et une coque supérieure.

La coque supérieure comprend une partie de liaison soudée à la coque inférieure et une partie formant capot qui est reliée à la partie de liaison par une charnière. Une bande de scellage est appliquée sur les bords de la fente définie entre le capot et la partie de liaison.

L'emballage comprend aussi une coiffe de protection permettant de couvrir la bande de scellage. L'opérateur peut enlever la coiffe pour pouvoir retirer la bande de scellage.

Une fois la bande de scellage retirée, la mobilité de la charnière de la coque supérieure est libérée de sorte que l'opérateur peut pivoter le capot de la coque supérieure, pour pouvoir extraire le contenu de l'emballage.

Il reste cependant souhaitable d'améliorer encore un tel emballage pour en faciliter son utilisation par un opérateur.

### RESUME DE L'INVENTION

A cet effet, l'invention a pour objet un emballage apte à contenir un objet, ledit emballage comprenant :
- un corps creux présentant une ouverture de sortie dudit objet ;
- un capot relié au corps creux par une charnière, permettant de fermer le corps creux,
- une coiffe de protection,

dans lequel ledit emballage comprend en outre un dispositif de liaison entre la coiffe de protection et le capot,
la coiffe de protection étant déplaçable entre :
   - une position de fermeture dans laquelle elle coiffe le capot et dans laquelle le dispositif de liaison présente une configuration réduite, et
   - une position d'ouverture dans laquelle elle est écartée du corps creux et dans laquelle le dispositif de liaison est dans une configuration déployée,
la coiffe de protection formant avec le dispositif de liaison, dans ladite position d'ouverture, un bras de levier couplé au capot.

L'emballage peut aussi comporter une ou plusieurs des caractéristiques suivantes prises dans toute combinaison techniquement admissible.

Selon une caractéristique avantageuse de l'invention, ledit emballage comprend un système de fermeture, retirable, défaisable ou cassable, permettant de maintenir le capot en position de fermeture du corps creux.

Selon une caractéristique avantageuse de l'invention, l'emballage comprend une bande de scellage qui est appliquée sur les bords d'une fente définie entre le capot et le corps creux.

Selon une caractéristique avantageuse de l'invention, la charnière présentant un axe et le corps creux présentant un axe de sortie dudit objet par ladite ouverture, le dispositif de liaison est rigide vis-à-vis d'un effort appliqué selon une direction transversale à l'axe de sortie du corps creux et transversale à l'axe de la charnière, pour permettre de faire pivoter le capot.

Selon une caractéristique avantageuse de l'invention, le dispositif de liaison comprend un système de maintien configuré pour, en position de fermeture de la coiffe de protection, maintenir le dispositif de liaison en configuration réduite, par exemple pliée ou contractée, tout en permettant une désactivation dudit système de maintien lors du passage de la coiffe de protection de ladite position de fermeture à ladite position d'ouverture qui entraine le passage du dispositif de liaison en configuration déployée.

Selon une caractéristique avantageuse de l'invention, le dispositif de liaison se présente sous la forme d'un corps présentant un contour fermé déformable, de préférence plastiquement, par traction.

Selon une caractéristique avantageuse de l'invention, le dispositif de liaison comprend deux bras, chaque bras présentant un coude.

Selon une caractéristique avantageuse de l'invention, chaque bras présente un amincissement de matière au niveau du coude. Selon un aspect particulier, des amincissements sont aussi prévus entre les bras et les traverses.

Selon une caractéristique avantageuse de l'invention, chaque bras présente un système d'accrochage configuré pour maintenir l'une contre l'autre les deux portions de bras qui s'étendent de part et d'autre du coude en position pliée du bras.

Selon une caractéristique avantageuse de l'invention, chaque bras présente un dispositif d'accrochage configuré pour, en coopération avec un dispositif d'accrochage correspondant ménagé sur le capot, maintenir ledit bras couplé au capot en configuration réduite du dispositif de liaison.

Selon une caractéristique avantageuse de l'invention, les deux bras étant reliés entre eux par une traverse inférieure et une traverse supérieure, le système de maintien comprend un organe mâle ménagé sur l'une des traverses et un organe femelle ménagé sur l'autre traverse, lesdits organes mâle et femelle étant configurés pour coopérer par encliquetage l'un avec l'autre en position de fermeture de la coiffe de protection.

Selon une caractéristique avantageuse de l'invention, le capot présente un logement d'insertion configuré pour recevoir et maintenir une partie du dispositif de liaison par rapport au capot.

Selon une caractéristique avantageuse de l'invention, ledit emballage comprend un système de couplage du dispositif de liaison au capot, le système de couplage étant configuré de sorte que, à l'état déployé du dispositif de liaison, le dispositif de liaison est pivotable par rapport au capot de manière à pouvoir amener la bordure de l'ouverture de la coiffe en appui sur le capot.

Selon une caractéristique avantageuse de l'invention, la coiffe de protection comprend deux parties de coiffe qui sont assemblables l'une avec l'autre, pour permettre de monter une partie du dispositif de liaison dans un logement d'insertion de l'une des parties de coiffe par une ouverture latérale de ladite partie de coiffe, et de refermer ladite ouverture latérale à l'aide de l'autre partie de coiffe.

Selon une caractéristique avantageuse de l'invention, l'emballage loge un emballage intérieur contenant un objet.

Selon une caractéristique avantageuse de l'invention, l'emballage loge un dispositif de préhension auquel est couplé ledit objet.

L'invention concerne également un procédé de déconditionnement d'un objet logé dans un emballage tel que décrit ci-dessus, dans lequel ledit procédé comprend les étapes suivantes :
- déplacement de la coiffe de protection en position d'ouverture, la coiffe de protection restant liée au capot par le dispositif de liaison ;
- application d'un effort sur la coiffe de protection pour faire pivoter le capot autour de l'axe de la charnière.

Selon une caractéristique avantageuse de l'invention, ledit procédé comprend en outre, entre lesdites étapes de déplacement de la coiffe de protection et d'application d'un effort sur la coiffe de protection pour faire pivoter le capot, une étape de pivotement de la coiffe de protection pour amener la bordure de l'ouverture de la coiffe de protection en appui sur le capot.

Selon une caractéristique avantageuse de l'invention, ledit procédé comprend en outre après l'étape de déplacement de la coiffe de protection, une étape de retrait d'une bande de scellage appliquée sur les bords d'une fente définie entre le capot et le premier corps creux.

Selon une caractéristique avantageuse de l'invention, le procédé comprend en outre une étape de séparation du capot par rapport au corps creux par application d'un effort de traction ou torsion sur le capot et/ou la coiffe de protection.

Selon une caractéristique avantageuse de l'invention, la coiffe de protection est éloignée d'une zone d'accès audit corps creux par pivotement ou détachement de l'ensemble du capot et de la coiffe par rapport au corps creux.

### BREVE DESCRIPTION DES DESSINS

D'autres caractéristiques et avantages de l'invention ressortiront encore de la description qui suit, laquelle est purement illustrative et non limitative et doit être lue en regard des dessins annexés, sur lesquels :
- la figure 1 est une vue éclatée d'un emballage conformément à un mode de réalisation de l'invention, le dispositif de liaison étant représenté en position déployée ;
- la figure 2 est une vue en perspective d'une partie d'un emballage conformément à un mode de réalisation de l'invention, montrant le couplage du dispositif de liaison au capot de la coque supérieure ; la coiffe n'étant pas représentée et le dispositif de liaison étant représenté en position déployée pour une meilleure lisibilité de la figure ;

- la figure 3 est une vue en perspective d'une partie d'un emballage conformément à un mode de réalisation de l'invention, montrant le couplage du dispositif de liaison à la coiffe de protection, le capot n'étant pas représenté et le dispositif de liaison étant en position déployée ;
- la figure 3A est une vue en perspective d'une partie d'un emballage conformément à un mode de réalisation de l'invention, montrant le couplage du dispositif de liaison à la coiffe de protection, le capot n'étant pas représenté et le dispositif de liaison étant en position réduite ;
- la figure 4 est une vue en perspective d'une coiffe de protection, réalisée en deux parties, d'un emballage conformément à un mode de réalisation de l'invention, à l'état non encore assemblé desdites parties de la coiffe de protection ;
- la figure 5 est une vue d'un dispositif de liaison, par exemple conforme à celui de la figure 2 ou 3, d'un emballage selon un mode de réalisation de l'invention, à l'état plié dudit dispositif de liaison ;
- la figure 6 est une vue partielle en perspective d'un emballage conformément à un mode de réalisation de l'invention, en position de fermeture de la coiffe de protection ;
- la figure 7 est une vue partielle en perspective de l'emballage de la figure 6, en position d'ouverture de la coiffe de protection de sorte que l'accès à la bande de scellage est libéré ;
- la figure 8 est une vue partielle en perspective de l'emballage de la figure 7, à l'état retiré de la bande de scellage par rapport à la coque supérieure sur laquelle ladite bande de scellage était appliquée ;
- la figure 9 est une vue partielle en perspective de l'emballage de la figure 8, à l'état ouvert par pivotement du capot de la coque supérieure ;
- la figure 10 est une vue partielle en perspective de l'emballage de la figure 9, à l'état cassé de la charnière par traction ;
- la figure 11 est une vue partielle en perspective de l'emballage de la figure 10, à l'état cassé de la charnière par torsion ;
- la figure 12 est une vue en perspective d'un capot et d'un dispositif de liaison (sans la coiffe) d'un emballage selon une variante de réalisation l'invention ;
- la figure 13 est une vue en perspective d'un capot et d'un dispositif de liaison (sans la coiffe) d'un emballage selon une autre variante de réalisation de l'invention, dans laquelle le dispositif de liaison est télescopique ;
- la figure 14 est une vue en perspective d'un exemple de préhenseur, auquel est couplé un objet, qui peut être contenu dans un emballage conforme à l'invention ;
- la figure 15 est une vue éclatée d'un emballage intérieur, contenant un objet, ledit emballage intérieur pouvant être contenu dans un emballage (extérieur) conforme à l'invention ;
- la figure 16 est une vue éclatée d'un emballage conformément à un autre mode de réalisation de l'invention, le dispositif de liaison étant représenté en position déployée ;
- la figure 17 est une vue avant assemblage d'une partie de la coiffe de l'emballage de la figure 16 avec le dispositif de liaison ;
- la figure 17A est une vue à l'état assemblé de la partie de coiffe et du dispositif de liaison de la figure 17 ;
- la figure 18 est une vue de face du dispositif de liaison de l'emballage de la figure 16 en position déployée ;
- la figure 18A est une vue en perspective du dispositif de liaison de la figure 18 en position pliée (réduite) ;
- la figure 19 est une vue en perspective du dispositif de liaison de l'emballage de la figure 16 en position pliée et couplée avec le capot ;
- la figure 20 est une vue partielle en perspective de dessus du capot montrant un système de couplage ménagé sur le capot de l'emballage de la figure 16 et destiné à coopérer avec un système correspondant ménagé sur le dispositif de liaison ;
- la figure 21 est une vue partielle en perspective du dispositif de liaison montrant un système de couplage ménagé sur le dispositif de liaison de l'emballage de la figure 16 et destiné à coopérer avec un système correspondant ménagé sur le capot ;
- les figures 22 à 27A illustrent des étapes d'ouverture d'un emballage conforme à celui de la figure 16.

### DESCRIPTION DETAILLEE

Le concept de l'invention est décrit plus complètement ci-après avec référence aux dessins joints, sur lesquels des modes de réalisation du concept de l'invention sont montrés. Des numéros similaires font référence à des éléments similaires sur tous les dessins. Cependant, ce concept de l'invention peut être mis en oeuvre sous de nombreuses formes différentes et ne devrait pas être interprété comme étant limité aux modes de réalisation exposés ici. Au lieu de cela, ces modes de réalisation sont proposés de sorte que cette description soit complète, et communiquent l'étendue du concept de l'invention aux hommes du métier. L'étendue de l'invention est par conséquent définie par les revendications jointes. Les modes de réalisation qui suivent sont examinés, par souci de simplification, en relation avec la terminologie et la structure d'un emballage.

Une référence dans toute la spécification à « un mode de réalisation » signifie qu'une fonctionnalité, une structure, ou une caractéristique particulière décrite en relation avec un mode de réalisation est incluse dans au moins un mode de réalisation de la présente invention. Ainsi, l'apparition de l'expression « dans un mode de réalisation » à divers emplacements dans toute la spécification ne fait pas nécessairement référence au même mode de réalisation. En outre, les fonctionnalités, les structures, ou les caractéristiques particulières peuvent être combinées de n'importe quelle manière appropriée dans un ou plusieurs modes de réalisation.

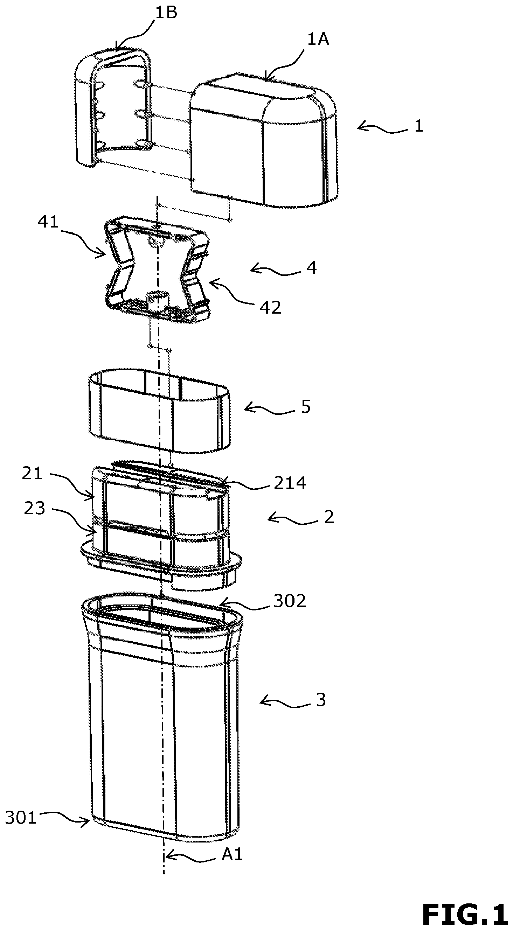

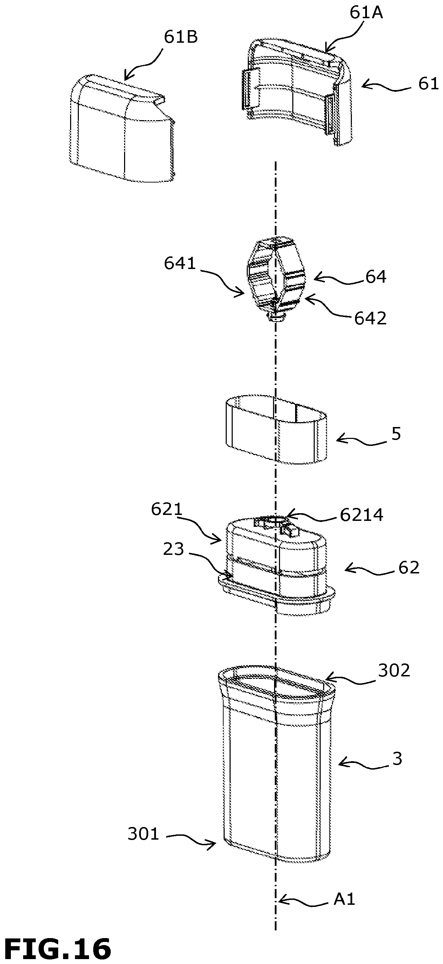

Selon des modes de réalisation et plus particulièrement en référence aux figures 1 à 15, il est proposé un emballage 6 comprenant une coque inférieure 3 et une coque supérieure 2. Il est également proposé un autre mode de réalisation aux figures 16 à 27A, qui propose un emballage 66 reprenant une partie des composants du mode de réalisation de la figure 1 et présentant des modifications sur d'autres composants. Ainsi, la coque inférieure 3, la bande de scellage 5 et la partie inférieure 23 de la coque supérieure 62 peuvent être identiques ou similaires dans le mode de réalisation des figures 1 et 16, de sorte que les mêmes numéros de référence leur sont affectés. Pour autant, comme détaillé ci-après, le capot 621 de la coque supérieure 62, le dispositif de liaison 64, et la coiffe 61 du mode de réalisation de la figure 16 diffèrent par rapport au composants correspondants 21, 4 et 1 du mode de réalisation de la figure 1.

La description réalisée ci-après de l'emballage 6 en lien avec le mode de réalisation des figures 1 à 15 peut s'appliquer à l'emballage 66, hormis bien entendu en ce qui concerne les éléments de l'emballage 66 qui différent de ceux de l'emballage 6. En particulier, la description réalisée pour le capot 21 peut aussi s'appliquer au capot 621 excepté en ce qui concerne la rainure 214 du capot 21 qui, pour le capot 621, est remplacée par les éléments de couplage 6214, 6217. De même, la description réalisée pour le dispositif de liaison 4 peut aussi s'appliquer au dispositif de liaison 64 excepté pour le système de couplage du dispositif de liaison 64 au capot 621 qui diffère du système de couplage du dispositif de liaison 4 au capot 21. De même, le couplage du dispositif de liaison 64 au capot 61 diffère de celui du dispositif de liaison 4 à la coiffe 1 et les bras du dispositif de liaison 4 se distinguent de ceux du dispositif de liaison 64.

Selon un mode de réalisation, la coque inférieure 3 et/ou la coque supérieure 2 sont réalisées en un matériau translucide, de préférence transparent.

La coque supérieure 2 comprend une partie de liaison 23 et un capot 21 reliés entre eux par une charnière 22. Selon un mode de réalisation, le capot 21, la charnière 22 et la partie de liaison 23 de la coque supérieure 2 sont formés d'une seule pièce, de préférence en plastique. La charnière 22 peut être formée par une simple zone de liaison entre le capot 21 et la partie de liaison 23. Selon un aspect particulier la charnière peut être cassée par torsion et/ou traction.

Chaque coque est considérée comme une coque ouverte au sens où elle forme une cavité borgne et permet à l'état assemblé avec l'autre de définir une chambre fermée à l'état appliqué d'une bande de scellage au niveau du capot 21 et de la partie de liaison 23, comme détaillé ci-après.

Dans l'exemple illustré aux figures, la bande de scellage forme un système de fermeture empêchant l'accès à l'intérieur du corps creux. La description réalisée ci-après dans le cas d'une bande de scellage s'applique aussi à d'autres types de système de fermeture. Ainsi, en variante, le système de fermeture peut être de la colle ou une soudure appliquée sur une zone entre le capot et le corps creux, ou encore le système de fermeture peut être une zone de liaison, fragilisée, par exemple en étant rendue sécable, entre le capot et le corps creux. Dans ces variantes, le système de fermeture se défait ou se rompt lors du pivotement du capot autour de l'axe de la charnière.

Ainsi, en position de fermeture, la coiffe 1 de protection coiffe le capot 21 et masque le système de fermeture. En position d'ouverture de la coiffe 1 de protection, le système de fermeture est accessible. Lorsque le capot 21 est coiffé par la coiffe 1, le capot 21 s'étend à l'intérieur de la coiffe au moins partiellement, de préférence complètement. En position d'ouverture de la coiffe 1 de protection, le capot 21 s'étend moins ou pas à l'intérieur de la coiffe 1 par rapport à la position de fermeture de la coiffe 1.

L'emballage 6 peut contenir un autre emballage, dit emballage intérieur. L'emballage intérieur peut contenir un implant médical. L'emballage intérieur et l'emballage extérieur forment alors un double-emballage.

En variante, on peut prévoir que l'emballage intérieur contienne un objet autre qu'un implant médical. L'emballage intérieur peut contenir d'autre(s) pièce(s) médicale(s) de préférence stérilisée(s).

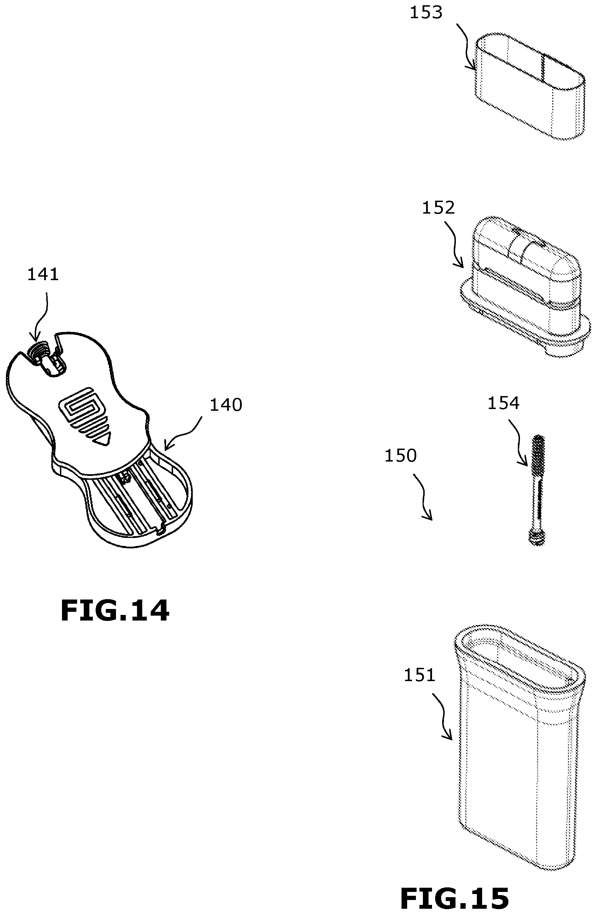

Comme illustré à la figure 15 qui représente un emballage intérieur 150, l'emballage intérieur peut comprendre une coque inférieure 151 et une coque supérieure 152 munie d'une bande de scellage 153 de manière similaire à celui de l'emballage 6, considéré alors comme emballage extérieur. L'emballage intérieur 150 contient un objet tel qu'une vis médicale 154.

En particulier l'emballage intérieur peut être similaire ou identique à un emballage intérieur tel que décrit dans la demande internationale WO2018078242A1, ou dans la demande internationale WO2019030451A2. Selon une variante de réalisation on peut prévoir que l'emballage intérieur soit similaire ou identique à l'emballage muni du dispositif de liaison décrit dans la présente demande, avec des dimensions adaptées pour loger dans l'emballage extérieur.

En variante, comme illustré plus particulièrement à la figure 16, on peut aussi prévoir que l'emballage extérieur loge un préhenseur 140 (ou système de maintien d'objet) associé à un objet, tel qu'une vis médicale 141.

A titre d'exemple le préhenseur (ou système de maintien d'objet) peut être similaire ou identique à celui ou ceux décrits dans la demande internationale WO2018065733A1, ou la demande internationale WO2019030451 A2, ou la demande internationale WO2019220067 A1.

On peut aussi prévoir que l'emballage 6 loge directement un objet souhaité, tel qu'une ou plusieurs pièce(s) médicale(s), sans emballage intérieur ou préhenseur intermédiaire.

L'objet peut être par exemple une pièce solide, telle qu'une vis pour chirurgie, ou tout autre type d'objet, en particulier tout autre type d'implant. En outre, ledit objet peut être un liquide ou une poudre. Ledit objet et, de préférence, les différentes parties de l'emballage, sont stérilisés par exemple par rayonnement.

L'emballage (ou le double emballage) est conçu à des fins de préservation du caractère stérile dudit objet et en vue du déballage de l'objet dans des conditions aseptiques ou proches de l'asepsie.

### Coque inférieure

La coque inférieure 3 se présente sous la forme d'un corps creux présentant une extrémité fermée formant fond 301 et une extrémité opposée 302 qui est ouverte. Selon un aspect particulier détaillé ci-après le pourtour de l'extrémité 302 est fixé à la partie de liaison 23 de la coque supérieure.

La coque inférieure 3 peut être similaire ou identique à celle décrite dans la demande WO2018078242A1.

### Coque supérieure

La coque supérieure 2 peut aussi être similaire ou identique à celle décrite dans la demande WO2018078242A1, à la différence que le capot 21 de la coque supérieure 2 est couplé au dispositif de liaison 4. A cet effet et comme proposé dans l'exemple illustré aux figures, la partie supérieure 10A du capot 1 peut être munie d'un système spécifique de réception du dispositif de liaison 4.

La partie de liaison 23 de la coque supérieure 2 se présente sous la forme d'un corps traversant. En particulier la partie de liaison 23 se présente sous la forme d'un tronçon de la coque supérieure. On peut prévoir que la partie de liaison 23 soit de forme générale cylindrique.

La charnière 22, qui relie une portion du pourtour supérieur de la partie de liaison 23 au pourtour du capot 21, permet d'articuler le capot 21 par rapport à la partie de liaison 23 entre une position relevée (ouverte) libérant l'accès à l'intérieur de la partie de liaison 23 et une position abaissée (fermée) empêchant l'accès à l'intérieur de ladite partie de liaison 23.

Selon un aspect préférentiel, une fois assemblées, ladite partie de liaison 23 de la coque supérieure 2 et la coque inférieure 3 forment deux pièces fixées l'une à l'autre de manière indémontable par l'utilisateur. Le pourtour inférieur de la partie de liaison 23 est emboité dans une portion d'extrémité évasée de la coque inférieure 3. La coque inférieure 3 est fixée à la partie de liaison 23, par exemple par soudage. Préférentiellement, la fixation est réalisée par soudage entre une collerette de la partie de liaison 23 et le pourtour de l'extrémité 302 de la coque inférieure 3.

En position fermée du capot, les pourtours du capot 21 et de la partie de liaison 23 sont situés en regard l'un de l'autre et forment ensemble ladite coque supérieure.

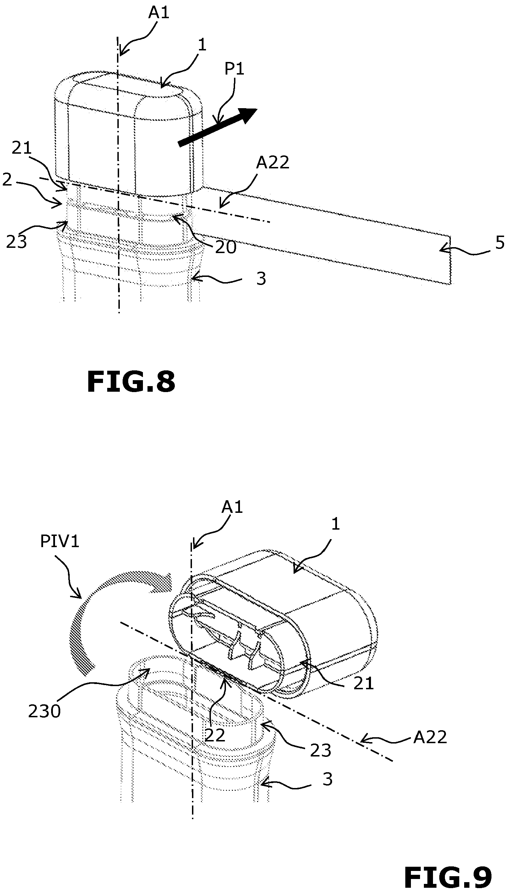

En position de fermeture du capot 21, celui-ci définit avec la partie de liaison 23 une fente 20 (visible en particulier à la figure 8) qui s'étend d'une extrémité de la charnière 22 à l'extrémité opposée de ladite charnière 22.

Dans les exemples illustrés aux figures et comme rappelé ci-dessus, la partie de liaison 23 est fixée à la coque inférieure 3 de sorte que l'ensemble de la partie de liaison 23 et la coque inférieure 3 forme un corps creux, encore appelé corps creux principal, permettant de loger un objet. En variante, on peut prévoir que le corps creux principal soit formé d'une seule pièce.

On définit l'axe A1 comme étant l'axe de sortie de l'objet (hors du corps creux principal), c'est-à-dire l'axe central de l'ouverture 230 de la partie de liaison 23. En position de fermeture du capot 21, cet axe A1 correspond aussi à la normale à l'embouchure de la coiffe 1. Par ailleurs, dans l'exemple illustré aux figures, cet axe A1 correspond à l'axe longitudinal de l'emballage.

Le corps creux principal présente ainsi à une de ses extrémités une ouverture correspondant à l'ouverture 230 (visible à la figure 9 en position ouverte du capot 21) dont l'accès est obturé en position de fermeture du capot 21. Dans l'exemple illustré aux figures, l'extrémité opposée du corps creux principal est fermée et forme le fond 301 dudit corps creux principal. L'objet logé dans l'emballage peut bien entendu dépasser en hauteur du corps creux principal et donc de la partie de liaison 23, jusqu'à l'intérieur de l'espace défini par le capot 21 (qui se présente sous la forme d'une coque dans les exemples illustrés aux figures).

La charnière 22 permet de pivoter le capot 21 entre ladite position de fermeture dans laquelle il ferme le corps creux principal 3, 23, c'est-à-dire où il empêche l'accès par ladite ouverture 230 du corps creux principal à l'intérieur dudit corps creux principal, et une position d'ouverture dans laquelle l'accès à l'intérieur du corps creux principal par ladite ouverture 230 est libéré, ce qui permet à un opérateur d'extraire le contenu de l'emballage 6.

Une bande de scellage 5 est appliquée sur les bords d'une fente 20 définie entre le capot 21 et la partie de liaison 23, ainsi que sur la charnière 22.

### Bande de scellage

La bande de scellage 5 relie, du côté extérieur de la coque supérieure 2, les bords du capot 21 et de la partie de liaison 23 qui se font face à l'état fermé (ou abaissé) du capot 21, sur toute la longueur des bords des coques de l'emballage et d'un bord à l'autre de manière à sceller l'emballage.

Préférentiellement, la bande de scellage 5 est appliquée par une de ses extrémités sur la charnière 22, s'étend le long des bords du capot 21 et de la partie de liaison 23, qui s'étendent en regard l'une de l'autre, et revient s'appliquer sur la charnière 22.

On obtient ainsi une très bonne étanchéité aux bactéries et au liquide, sans discontinuité le long de la zone d'ouverture de la coque supérieure.

Préférentiellement, la bande de scellage 5 est une bande pelable. Une fois la bande appliquée, celle-ci peut être retirée en tirant dessus pour la peler. On entend par pelable le fait que la bande ne se déchire pas lorsqu'on la retire, tandis que la colle reste sur la zone des coques précédemment scellée.

Selon un exemple de réalisation, la bande de scellage peut être une bande thermocollante par exemple en matériau synthétique non-tissé fabriquée à partir de fibres de polyéthylène, usuellement vendue sous la marque déposée TYVEK.

### Coiffe de protection

Une coiffe 1 de protection permet de coiffer le capot 21 lorsque ladite coiffe est dans une position, dite position de fermeture, dans laquelle elle masque la bande de scellage 5. La coiffe peut être déplacée, par exemple levée selon l'axe A1, en une position écartée de la coque inférieure 3 pour permettre l'accès à la bande de scellage 5.

En position de fermeture de la coiffe 1 de protection, la coiffe 1 de protection est proche ou en contact de la coque inférieure 3.

Comme expliqué ci-après, la coiffe 1 de protection peut être réalisée en deux parties 1A, 1B destinées à être assemblées l'une à l'autre.

### Dispositif de liaison

Un dispositif de liaison 4 est situé entre la coiffe 1 de protection et le capot 21 de la coque supérieure 2. Le dispositif de liaison 4 s'étend à l'intérieur de la coiffe 1.

Le dispositif de liaison 4 est configuré pour maintenir la coiffe 1 de protection liée au capot 21 quelle que soit la position de la coiffe 1 de protection.

Le dispositif de liaison 4 permet, en position d'ouverture de la coiffe 1 de protection, de maintenir la coiffe de protection couplée au capot. Autrement dit, la coiffe peut être levée par rapport au capot 21 et donc à la coque inférieure 3, tout en restant liée au capot 21.

Lorsque la coiffe 1 de protection est en position d'ouverture, l'extrémité supérieure de la coiffe 1 de protection est écartée du capot 21, par comparaison avec la position de fermeture de la coiffe 1 de protection où l'extrémité supérieure de la coiffe 1 de protection est alors en position plus proche du capot 21.

Dans la position d'ouverture de la coiffe 1 de protection, l'ensemble de la coiffe 1 de protection et du dispositif de liaison 4 qui restent liés l'un à l'autre, forme un bras de levier couplé au capot 21 qui facilite l'ouverture du capot 21. Ce bras de levier est fixé au capot 21 par la liaison entre le dispositif de liaison 4 et le capot 21, et l'extrémité libre de ce bras de levier est formée par l'extrémité supérieure de la coiffe 1 de protection. Un tel bras de levier couplé au capot 21 facilite ainsi l'ouverture du capot 21 par pivotement du capot 21 et du bras de levier autour de l'axe A22 de la charnière 22.

Le dispositif de liaison 4 est rigide vis-à-vis d'un effort appliqué sur le dispositif de liaison 4 pour permettre le pivotement de la coiffe et dudit dispositif de liaison selon une direction transversale à l'axe A1 et à l'axe A22 de la charnière 22. On entend par rigide le fait que la structure du dispositif de liaison 4 est adaptée pour transmettre au capot 21, tout ou partie d'un effort appliqué au dispositif de liaison 4 (directement ou indirectement) selon une direction transversale à l'axe A1 et à l'axe A22 de la charnière 22, ou selon une direction transversale au plan moyen dans lequel s'étendent les bras du dispositif de liaison, par exemple une direction parallèle à l'axe de pliage de chaque bras, pour faire pivoter le capot 21 autour de l'axe de la charnière.

En particulier, dans les exemples illustrés aux figures, le dispositif de liaison est rigide vis-à-vis d'un effort qui lui est appliqué selon une direction transversale (ou orthogonale) à son plan moyen pour entrainer un pivotement du capot 21 autour de l'axe A22 de la charnière 22.

En effet, la rigidité du dispositif de liaison 4, vis-à-vis d'un effort qui lui est appliqué (par l'intermédiaire de la coiffe 1 de protection sur laquelle l'opérateur exerce l'effort P1 comme schématisé à la figure 8) qui tend à ouvrir le capot 21, permet de transmettre cet effort au capot 21 et donc de le faire pivoter autour de l'axe A22 la charnière 22.

On peut prévoir que le dispositif de liaison 4 soit réalisé par un procédé d'injection plastique en une position intermédiaire entre la position déployée et la position pliée ou contractée, avec par exemple un plan de joint passant par le plan moyen du dispositif de liaison 4.

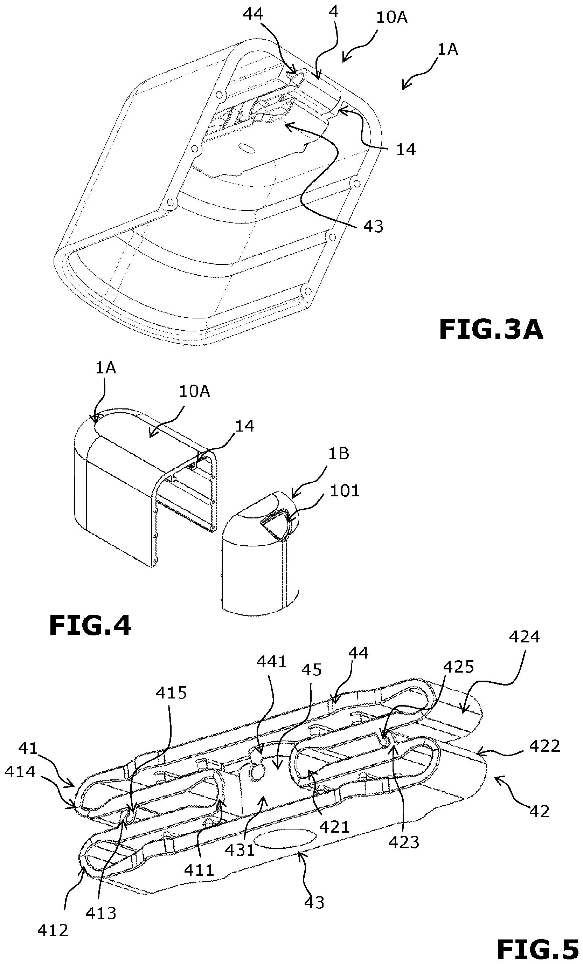

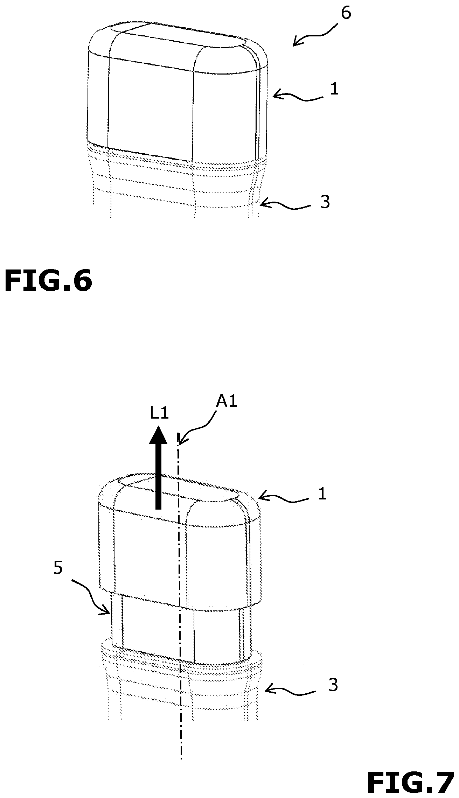

En position de fermeture de la coiffe 1 de protection (Figure 6), le dispositif de liaison 4 est dans une configuration de dimension réduite selon l'axe A1, par comparaison avec sa dimension lorsque la coiffe 1 est en position d'ouverture. Dans l'exemple illustré en particulier à la figure 5, on voit ainsi que le dispositif de liaison 4 est plié ou contracté.

Dans ladite position de fermeture, la coiffe 1 de protection couvre la bande 5 de scellage ce qui permet de la protéger.

En position d'ouverture de la coiffe 1 de protection (Figure 7), le dispositif de liaison 4 est dans une configuration de dimension selon l'axe A1 plus grande par comparaison avec sa dimension lorsque la coiffe est en position de fermeture.

Dans l'exemple illustré en particulier à la figure 7 ou à la figure 8, le dispositif de liaison 4 (non visible à cause de la coiffe de protection) est déployé. En position d'ouverture de la coiffe 1 de protection (c'est-à-dire en position levée de la coiffe par rapport à la coque inférieure) l'accès à la bande de scellage est libéré, ce qui permet à un opérateur de la retirer.

Dans le mode de réalisation des figures 16 à 27A, le dispositif de liaison 64 est configuré pour, en position d'ouverture (levée) de la coiffe 61, permettre un pivotement du dispositif de liaison 64 d'un angle donné, de préférence de 90°, de manière à amener la bordure 612 de l'ouverture de la coiffe 61 dans une position stable en appui sur le dessus du capot 621. Le pivotement du dispositif de liaison 64 est un pivotement autour d'un axe confondu ou parallèle à l'axe A1 longitudinal de l'emballage. Cette mobilité de pivotement est rendue possible par le système de couplage qui comprend un pion 6432 et une bague 6214 et qui permet, à l'état déployé du dispositif de liaison 64 de sorte que les éléments d'accrochage 6413, 6423 sont libérés des éléments d'accrochages 6217 correspondants, de laisser le pion 6432 (qui est solidaire du dispositif de liaison 64) pivoter par rapport à la bague 6214 qui est solidaire du capot 621.

Cette position pivotée du dispositif de liaison 64 et donc de la coiffe 61, qui reste couplée au dispositif de liaison 64 lui-même couplé au capot 621, permet de maintenir la coiffe 61 au-dessus du capot 621, et donc de bénéficier d'une longueur importante et stable de bras de levier.

### Bras du dispositif de liaison

Dans l'exemple illustré aux figures 1 à 15, le dispositif de liaison 4 comprend deux bras 41, 42. Les deux bras sont reliés entre eux par une traverse inférieure 43 et une traverse supérieure 44. Préférentiellement, chaque traverse comprend une nervure de rigidification. Préférentiellement le dispositif de liaison est à l'état réduit (replié ou comprimé) en position initiale.

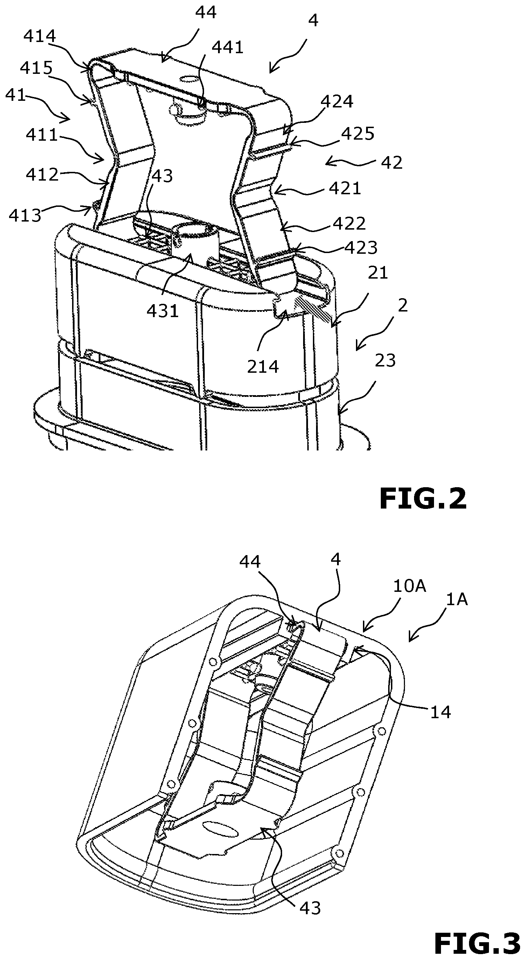

Dans l'exemple illustré plus particulièrement aux figures 2 et 5, chaque bras 41, 42 présente un coude 411, 421. Autrement dit, chaque bras 41, 42 présente une forme générale en V. Selon un aspect particulier le coude est situé vers l'intérieur du dispositif de liaison 4, c'est-à-dire vers l'intérieur du cadre déformable formé par les bras 41, 42 et les traverses 43, 44 du dispositif de liaison.

Les bras 41, 42 sont pliables et dépliables au niveau de leur coude 411, 412. Le dispositif de liaison 4 est configuré pour permettre une déformation symétrique des deux bras 41, 42 permettant de passer d'une position dépliée à une position pliée tout en gardant une rigidité du dispositif de liaison 4 vis-à-vis d'un effort qui serait exercé sur le dispositif de liaison 4 selon une direction transversale à l'axe A1 et à l'axe A22 (ou encore selon une direction transversale au plan moyen passant par les bras 41,42).

Selon un aspect particulier, chaque bras 41, 42 peut présenter un amincissement de matière au niveau du coude 411, 421 pour former des amorces de pliage facilitant le passage du dispositif de liaison 4 de sa position pliée ou contractée à sa position dépliée ou déployée, et inversement. En variante, il pourrait s'agir aussi d'un évidement de matière.

Selon un mode de réalisation, comme illustré plus particulièrement aux figures 2 et 5, chaque bras 41, respectivement 42, présente un système d'accrochage 413, 415, respectivement 423, 425, configuré pour maintenir l'une contre l'autre les deux portions 422, 424, respectivement 412, 414, de bras 42 qui s'étendent de part et d'autre du coude 421, respectivement 411, en position pliée du bras 42, respectivement 41.

Dans l'exemple illustré plus particulièrement aux figures 2 et 5, le système d'accrochage comprend ainsi deux crochets 413, 415 pour le bras 41 et deux crochets 423, 425 pour le bras 42 aptes à coopérer l'un avec l'autre. Les crochets sont répartis de part et d'autre du coude du bras correspondant.

Les crochets d'un bras coopèrent l'un avec l'autre lors du pliage du bras correspondant. Les crochets sont désengageables l'un par rapport à l'autre par déformation élastique lorsque le dispositif de liaison 4 subit un effort axial (selon l'axe A1) supérieur à une valeur seuil qui entraine le dépliage du bras correspondant.

Un tel système d'accrochage permet de limiter le risque de déformation des traverses du dispositif de liaison.

Dans le mode de réalisation des figures 16 à 27A où le dispositif de liaison présente des bras 641, 642, le système d'accrochage décrit ci-dessus est de préférence remplacé par des éléments d'accrochage 6413, 6423 destinés à s'encliqueter dans des logements correspondants 6217 ménagés sur le capot 621.

Les éléments d'accrochages 6413, 6423 sont désengageables des logements 6217 par déformation élastique lorsque le dispositif de liaison 64 subit un effort axial (selon l'axe A1) supérieur à une valeur seuil qui entraine le dépliage des bras 641, 642 correspondants.

Dans l'exemple des figures 16 à 27A, les bras 641 et 642 qui relient entre elles les traverses 643 et 644 sont coudés vers l'extérieur du dispositif de liaison 64, c'est-à-dire vers l'extérieur du cadre déformable formé par les bras et les traverses du dispositif de liaison.

Selon un aspect particulier et de manière similaire à ce qui a été décrit pour le dispositif de liaison 4, les bras du dispositif de liaison 64 présentent aussi des amincissements ou évidements de matière de manière à former des amorces de pliage facilitant le passage du dispositif de liaison de sa position pliée ou contractée à sa position dépliée ou déployée, et inversement.

### Système de maintien

Le dispositif de liaison 4 comprend un système de maintien 45 qui permet de maintenir le dispositif de liaison 4 dans la configuration pliée ou contractée, correspondant à la position de fermeture de la coiffe 1 de protection. Le dispositif de liaison 4 permet ainsi de maintenir la coiffe de protection en position fermée.

La fonction de maintien du système de maintien 45 reste cependant désactivable lors du passage de la coiffe 1 de protection de sa position de fermeture à sa position d'ouverture pour permettre le déploiement du dispositif de liaison 4.

Dans l'exemple illustré plus particulièrement aux figures 2 et 5, le système de centrage 45 comprend un pion 441 dont est muni l'une 44 des traverses qui est apte à coopérer avec un logement (ou fût) 431 correspondant dont est muni l'autre traverse 43.

Ainsi, en position fermée de la coiffe 1 de fermeture, c'est-à-dire en position rapprochée de la coiffe par rapport à la coque inférieure, le pion 441 est engagé par encliquetage dans le logement (ou fût) 431, de sorte que les traverses 43 et 44 sont maintenues en position rapprochées l'une de l'autre.

Les organes mâle 441 et femelle 431 du système de maintien des traverses 43 et 44 restent désengageables l'une par rapport à l'autre par déformation élastique (désencliquetage), lorsque le dispositif de liaison 4 subit un effort axial (correspondant à l'effort pour déplacer la coiffe de protection en position d'ouverture) qui est supérieur à une valeur seuil qui entraine l'écartement des traverses 43, 44 l'une par rapport à l'autre.

Selon un aspect particulier, le dispositif de liaison est maintenu centré par rapport au capot 21 et/ou la coiffe 1. Dans l'exemple illustré aux figures, le système de maintien 45 permet un tel centrage du dispositif de liaison 4 par rapport à la coiffe et/ou au capot 21. Ainsi, selon un aspect particulier, le pion 441 et le logement 431 sont centrés sur les traverses. Il en résulte que, à l'état activé du système de maintien 45, les traverses 44 et 43 sont centrées l'une sur l'autre.

En particulier, lors de l'ouverture de la coiffe 1 de protection qui reste liée au capot 21 par le dispositif de liaison 4, la coiffe 1 de protection tire sur la traverse 44 supérieure du dispositif de liaison 4, ce qui détache les deux organes 431, 441 du système de maintien 45 l'un par rapport à l'autre. Lors du déplacement de la coiffe 1 de protection, celle-ci reste fixée à la traverse 44 supérieure du dispositif de liaison 4, et la traverse 43 inférieure du dispositif de liaison 4 reste fixée au capot 21.

Selon des modes de réalisation et comme visible dans l'exemple illustré aux figures 2 et 5, le dispositif de liaison 4 conserve en position pliée ou contractée sensiblement un même axe de symétrie que celui qu'il présente en position dépliée ou déployée.

On peut prévoir une répartition inversée de l'élément mâle (pion) et de l'élément femelle (logement) sur les traverses. Ainsi dans l'exemple illustré aux figures 16 à 27A, le dispositif de liaison 64 est muni d'un système de maintien 645 qui comprend un pion 6431 dont est muni l'une 643 des traverses (celle destinée à être couplée au capot 621) qui est apte à coopérer avec un logement (ou fût) 6441 correspondant dont est muni l'autre traverse 644 (celle destinée à être couplée à la coiffe).

### Montage du dispositif de liaison

Selon un aspect particulier illustré aux figures 1 et 2, le capot 21 présente un logement d'insertion 214, qui est de préférence intégré dans la hauteur du capot 21. Le logement d'insertion 214 est ménagé sur la face externe de sa paroi supérieure.

Dans l'exemple illustré, le logement 214 est formé par une rainure dont la section délimite un passage en forme générale de T ou queue d'aronde.

Avantageusement, la rainure est débouchante à au moins une extrémité pour permettre une insertion latérale de la traverse 43 du dispositif de liaison 4 dans la rainure (voir figure 2), par déplacement relatif entre le dispositif de liaison 4 et le capot 21. En variante, le logement d'insertion 214 peut être formé par une réservation, munie de clips de retenue, configurée pour recevoir et maintenir la traverse 43 du dispositif de liaison 4 par rapport au capot 21.

La coiffe 1 présente aussi un logement d'insertion 14 destiné à recevoir la traverse supérieure 44 du dispositif de liaison 4. Le logement d'insertion 14 est ménagé dans la face intérieure de la partie supérieure de la coiffe 1.

En particulier, la coiffe 1 comprend deux parties 1A, 1B assemblables l'une avec l'autre, pour permettre de monter le dispositif de liaison 4 (en particulier la traverse 44) dans le logement d'insertion 14 par une ouverture latérale de la partie 1A qui est munie dudit logement d'insertion 14. La partie 1B permet de refermer l'ouverture latérale de la partie 1A de la coiffe de protection 1.

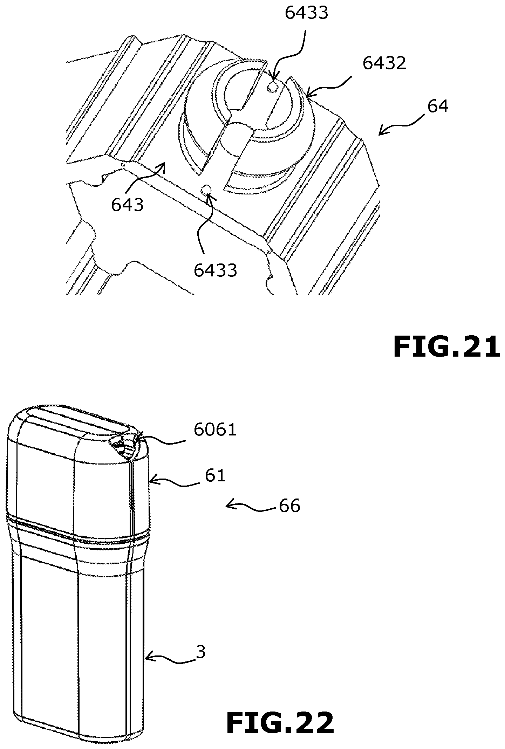

La partie 1B présente un orifice 101 pour permettre une prise en main aisée d'un film plastique thermo-rétractable qui peut être mis autour de l'emballage pour le protéger de la poussière, ou pour permettre les échanges gazeux lors d'une stérilisation par gaz, du type oxyde d'éthylène. Dans le mode de réalisation de la figure 22, la coiffe 61 comprend aussi un orifice similaire référencé 6061.

En variante, le logement d'insertion 14 peut être formé par une réservation, munie de clips de retenue, configurée pour recevoir et maintenir la traverse 44 du dispositif de liaison 4 par rapport à la coiffe 1 de protection.

En variante, on peut prévoir que le dispositif de liaison 4 soit formé d'une seule pièce avec le capot 21 ou la coiffe 1 de protection.

Le dispositif de liaison 4 est fixé à la partie 1A de la coiffe 1 de protection par déplacement relatif de l'un par rapport à l'autre. Préférentiellement, l'assemblage du dispositif de liaison 4 avec le capot 21 est réalisé avant application de la bande de scellage 5 sur la fente 20.

En particulier, on peut prévoir d'assembler le dispositif de liaison 4 avec le capot 21, d'appliquer la bande de scellage 5 sur le capot 21, puis d'assembler la partie 1A de la coiffe 1 de protection avec le dispositif de liaison 4, et enfin de fermer la partie 1A de la coiffe 1 de protection avec la partie 1B de la coiffe de protection.

En variante, le dispositif de liaison 4 peut être assemblé avec le capot 21 après application de la bande de scellage 5 sur la fente 20.

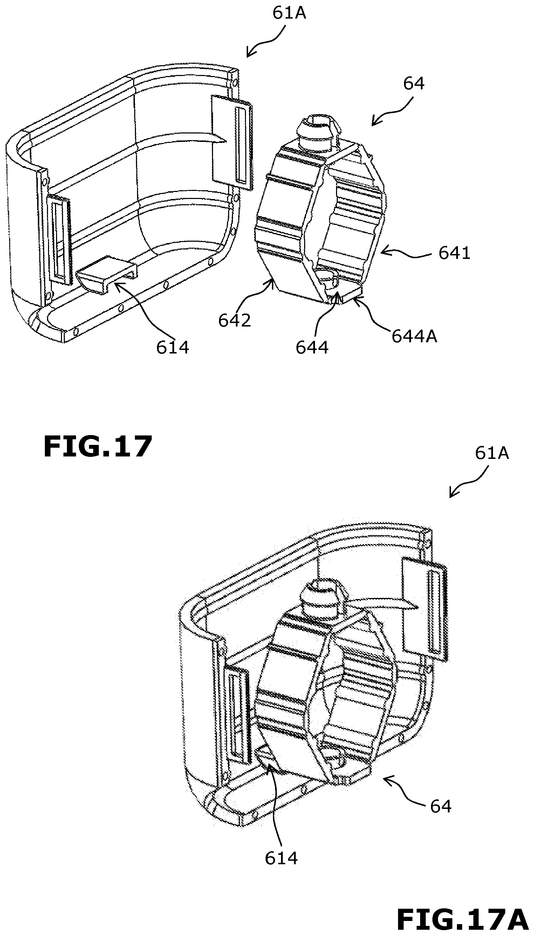

Dans le mode de réalisation des figures 16 à 27A, le système spécifique de réception du dispositif de liaison 64 dans la coiffe 61 comprend des logements 614 de réception ménagés dans les deux parties 61A, 61B de la coiffe (voir figures 17 et 17A). Ces logements 614 sont aptes à recevoir chacun une ailette 644A (encore appelée ailette d'assemblage) du dispositif de liaison 64. Dans l'exemple de la figure 16, chaque ailette est formée par une portion d'extrémité de la traverse 644 du dispositif de liaison 64.

Dans l'exemple de la figure 16, la coiffe 61 de protection est réalisée en deux parties, de préférence sensiblement symétriques, avec un plan d'assemblage médian qui s'étend selon la longueur de la coiffe 61 et perpendiculairement au plan d'ouverture de cette coiffe.

Comme rappelé ci-dessus, le dispositif de liaison 64 comprend des ailettes d'assemblage qui sont disposées orthogonalement au plan d'assemblage des deux parties de la coiffe 61, à l'état assemblé du dispositif de liaison avec lesdites parties de la coiffe 61. Ces ailettes d'assemblage sont disposées selon un axe qui est parallèle aux axes de pliage/dépliage des bras 641, 642 du dispositif de liaison 64. Le dispositif de liaison 4 présente un plan médian dans lequel s'étend les deux bras 641, 642. Les ailettes d'assemblage sont disposées de part et d'autre de ce plan médian.

Selon un mode de réalisation, le dispositif de liaison passe de la position réduite à la position déployée par déformation plastique afin d'éviter ou de limiter un effet de rappel élastique en position réduite.

Dans les exemples illustrés aux figures, le dispositif de liaison se présente sous la forme d'un corps présentant un contour fermé déformable. En position dépliée ou déployée, le contour fermé définit un passage traversant.

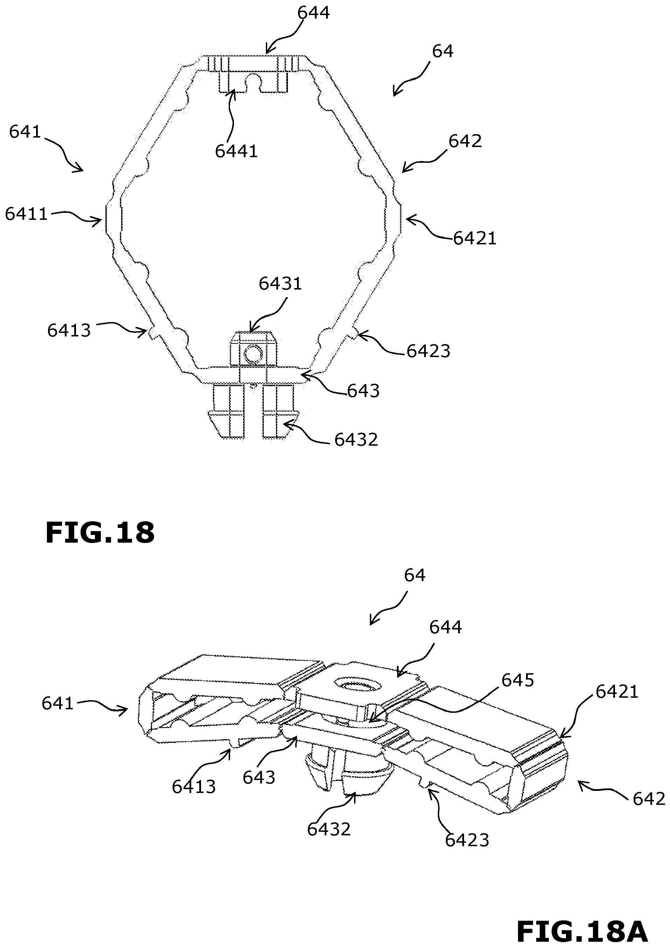

Dans le mode de réalisation des figures 16 à 27A, le dispositif de liaison 64 est couplé au capot 621 par un système de couplage, de préférence par encliquetage, d'un élément mâle, tel qu'un pion 6432, de préférence ménagé sur la traverse 643 du côté extérieur, dans un élément femelle, tel qu'un logement par exemple formé par une bague 6214, de préférence ménagé sur le capot 621. Dans l'exemple de la figure 18, le pion 6432 est fendu axialement et présente une extrémité tronconique adaptée à une insertion avec maintien dans la bague 6214.

Le système de couplage 6432/6214, une fois activé, s'oppose à un retrait du dispositif de liaison 64 par rapport au capot 621 par traction, mais permet, à l'état déployé du dispositif de liaison 64, un pivotement du dispositif de liaison 64 par rapport au capot 621 autour d'un axe confondu ou parallèle à l'axe A1, de préférence selon une plage angulaire donnée.

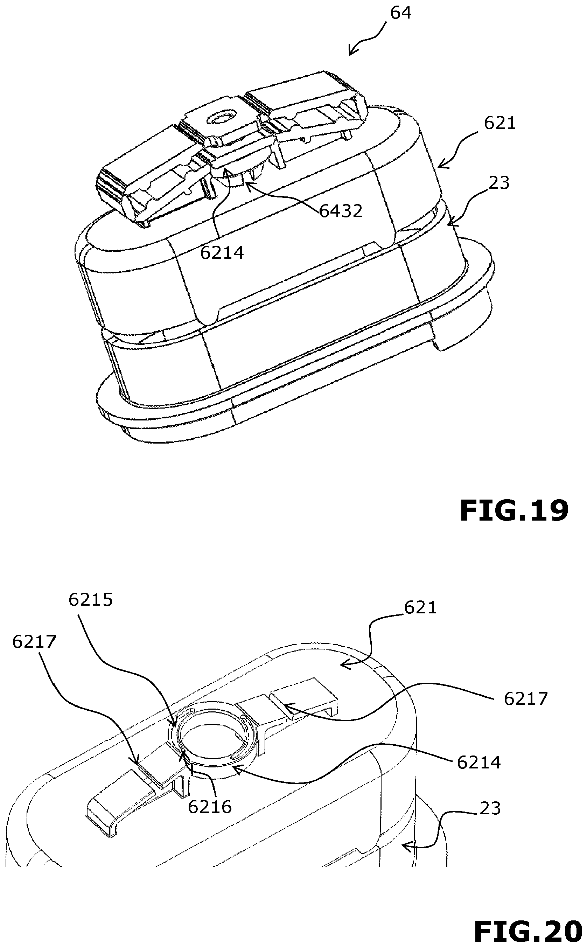

Dans l'exemple de la figure 20, la bague 6214 comprend sur sa face supérieure des rails 6215 formés par des gorges courbes qui sont aptes à recevoir des ergots 6433 associés au pion 6432 et ménagés sur le dispositif de liaison 64. Les rails 6215 permettent de guider un mouvement de pivotement du dispositif de liaison 64 par rapport au capot 621 par coopération des ergots 6433 du dispositif de liaison 64 dans les rails 6215 de l'élément femelle du capot 621.

Des moyens de maintien sont prévus pour maintenir le dispositif de liaison 64 en une position tournée d'un angle donné par rapport au capot 621, de préférence un angle de 90°. En particulier, dans l'exemple illustré à la figure 20, chaque rail comprend au niveau d'une extrémité un système de maintien de l'ergot, par exemple formé par un logement 6216 présentant un rétrécissement local du rail avant l'extrémité, de sorte que, une fois passé ce rétrécissement, l'ergot 6433 est maintenu à cette extrémité du rail 6215.

Selon un aspect particulier, chaque bras 641, 642 présente un élément d'accrochage 6413, 6423 configuré pour, en position pliée du dispositif de liaison 64, coopérer avec un élément d'accrochage complémentaire 6217, tel qu'une fente ménagée dans un élément de contre-forme présent sur le capot 621. Ces éléments d'accrochage 6413, 6423, 6217 permettent de maintenir le bras correspondant contre le capot.

En configuration fermée de la coiffe 61, la coiffe 61 recouvre majoritairement, de préférence complètement, le capot 621, et le dispositif de liaison 64 est en configuration pliée.

Lorsque l'opérateur tire sur la coiffe 61 selon l'axe A1, la coiffe 61 est amenée en configuration levée par rapport au capot 621 en entrainant le dispositif de liaison 64 en position déployée. Dans cette configuration simplement levée de la coiffe 61 (non encore pivotée), en projection selon l'axe A1 et dans un plan orthogonal à l'axe A1, le contour extérieur du capot 621 reste à l'intérieur du contour extérieur de la coiffe 61.

En configuration levée et pivotée d'un angle donné, par exemple 90°, de la coiffe 61 par rapport au capot 421 autour de l'axe A1, la bordure 612 de l'ouverture (pourtour inférieur) de la coiffe 61 est positionnée en appui sur le capot 621, ce qui maintient la coiffe 61 en position levée par rapport au capot 621 en empêchant une redescente de la coiffe 61.

Dans cette configuration levée et pivotée de la coiffe 61, en projection selon l'axe A1 et dans un plan orthogonal à l'axe A1, le contour extérieur du capot 621 s'étend en partie en dehors du contour extérieur de la coiffe 61.

Cette configuration levée pivotée de la coiffe 61 (qui entraine aussi le dispositif de liaison en configuration déployée et pivotée), permet ainsi de maintenir dégagé l'accès pour l'utilisateur à la coque supérieure 62, et en particulier à la bande de scellage 5. La bande de scellage peut alors être aisément retirée. Le pivotement de la coiffe 61 au-dessus du capot 621 permet d'obtenir une hauteur maximum et stable de la coiffe 61, ce qui permet de former un bras de levier efficace pour l'ouverture du capot 621 par pivotement de l'ensemble de la coiffe 61 et du capot 621 par rapport à la partie de liaison 23 de la coque supérieure 62 autour de l'axe A22 de la charnière 22 correspondante.

### Procédé de déconditionnement

Les figures 6 à 11 décrivent différentes étapes d'une séquence d'ouverture de l'emballage 6.

Initialement et comme illustré à la figure 6, la coiffe 1 de protection est en position fermée. La coiffe 1 de protection est proche de la coque inférieure 3 et couvre la bande de scellage 5.

L'opérateur tire avec un effort correspondant L1 (figure 7) sur la coiffe 1 de protection de sorte que la coiffe 1 de protection se lève par rapport à la coque inférieure 3. La coiffe 1 de protection est ainsi en position d'ouverture. L'effort L1 est exercé selon l'axe A1 (axe longitudinal de l'emballage).

L'effort L1 permet de désactiver le système de maintien 45 (figure 5) en tirant sur la traverse 44 du dispositif de liaison 4 qui est fixée à l'intérieur de la coiffe 1 de protection, ce qui désengage les organes mâle 441 et femelle 431 l'un par rapport à l'autre. Le système d'accrochage des bras 41, 42 est également désactivé par cet effort L1 de sorte que le dispositif de liaison 4 accompagne le déplacement de la coiffe 1 en dépliant ses bras 41, 42. Préférentiellement, le dispositif de liaison 4 subit une déformation plastique, en particulier au niveau des bras, lors du passage du système formé par les bras repliés à l'état initial, à l'état déployé.

La bande de scellage 5 est ainsi découverte et peut être retirée comme illustré à la figure 8. La charnière 22 qui relie le capot 21 à la partie de liaison 23 (et donc à la coque inférieure 3) n'est alors plus contrainte par la bande de scellage.

L'opérateur peut ainsi appliquer sur la coiffe 1 de protection un effort schématisé P1 sur la figure 8 pour faire pivoter le capot 21 autour de l'axe A22 de la charnière 22. En particulier, cet effort P1 est un effort transversal à l'axe A1 de l'emballage et à l'axe A22 de la charnière qui est transmis par la coiffe 1 de protection jusqu'au capot 21 par le dispositif de liaison 4 dont la structure le rend rigide vis-à-vis d'un tel effort. On peut aussi noter que l'effort P1 est transversal au plan moyen passant par les bras du dispositif de liaison.

Le dispositif de liaison 4 et la coiffe 1 de protection forment ainsi, en position d'ouverture, un bras de levier sur le capot 21. L'effort P1 appliqué à ce bras de levier entraine le pivotement PIV1 du capot 21 schématisé à la figure 9.

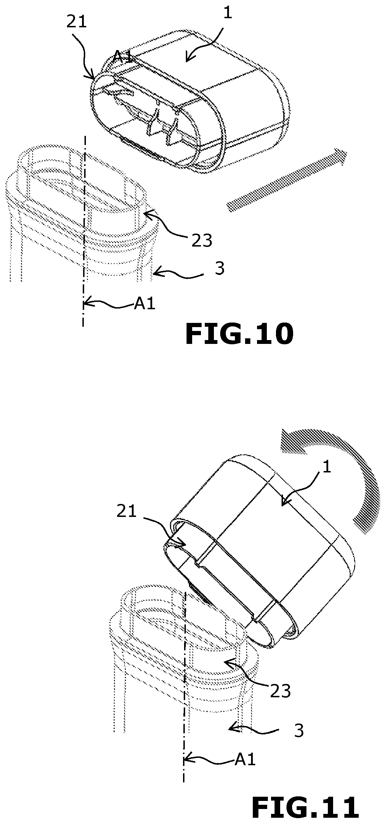

L'opérateur peut, comme illustré à la figure 10, détacher le capot 21, qui est pivoté en position ouverte, par rapport à la partie de liaison 23, en tirant sur le capot ou la coiffe jusqu'à la rupture de la charnière. En variante et comme illustré à la figure 11, l'opérateur peut séparer le capot 21, qui est pivoté en position ouverte, par rapport à la partie de liaison 23, par un mouvement de torsion en tournant le capot ou la coiffe autour d'un axe transversal à la charnière jusqu'à la rupture de la charnière.

L'opérateur peut alors extraire l'objet contenu dans le corps creux principal restant, qui correspond à la coque inférieure 3 et à la partie de liaison 23. On peut aussi prévoir que l'opérateur extrait l'objet contenu sans casser la charnière 22.

Comme rappelé ci-dessus le contenu extrait peut être un autre emballage (emballage interne), et/ou un préhenseur couplé à un objet (encore appelé système de maintien d'objet) ou encore l'objet lui-même.

Les figures 22 à 27A décrivent différentes étapes d'une séquence d'ouverture de l'emballage 66. Les étapes décrites ci-dessus pour l'emballage 6 sont applicables à l'emballage 66 avec en outre une étape de pivotement de la coiffe 61 pour la maintenir en position levée au-dessus et en appui du capot 621.

Initialement comme représenté à la figure 22, la coiffe 61 de l'emballage 66 est en position fermée, le dispositif de liaison 64 étant en configuration pliée (réduite). Le système de maintien 645 est actif et les bras 641, 642 sont maintenus couplés au capot 621 par coopération des éléments d'accrochage 6413, 6423 avec les éléments 6217 correspondants.

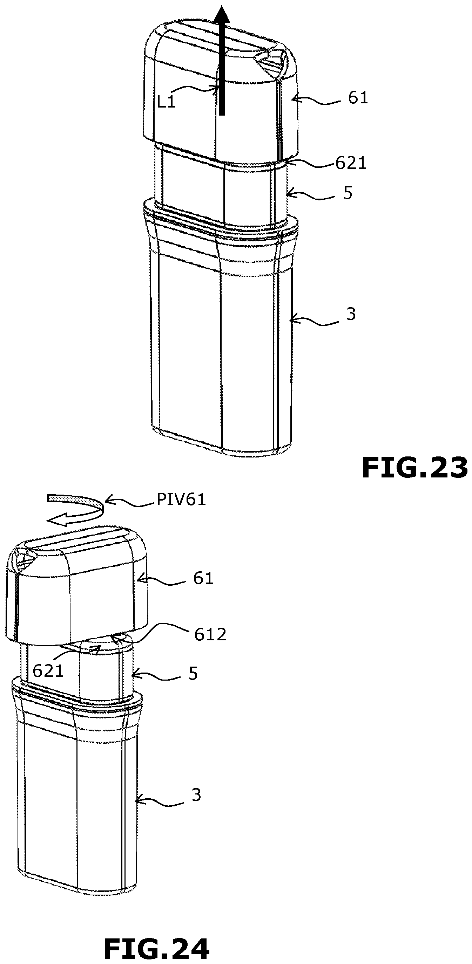

Comme illustré à la figure 23, pour ouvrir l'emballage 66, l'utilisateur tire sur la coiffe 61 pour l'écarter de la coque inférieure 3, et découvrir ainsi la bande de scellage 5. L'utilisateur exerce ainsi un effort sur la coiffe 61 selon une direction L1 parallèle à l'axe A1. Le déplacement de la coiffe 61 entraine le déploiement du dispositif de liaison 64 en désactivant le système de maintien 645 et le couplage entre eux des éléments d'accrochage 6413, 6423, 6217.

Préférentiellement et comme illustré à la figure 24, l'utilisateur pivote (flèche PIV61) la coiffe 61 autour d'un axe parallèle ou confondu avec l'axe A1, grâce à la liaison de pivotement offerte par le système de couplage (pion 6432 / bague 6214) entre le dispositif de liaison 64 et le capot 621. L'utilisateur peut ainsi orienter la coiffe 61 transversalement, de préférence orthogonalement, au capot 621 pour amener la bordure de l'ouverture de la coiffe 61 en appui sur le dessus du capot 621 et ainsi maintenir la distance entre la coiffe 61 et la coque inférieure 3, ce qui permet non seulement de pouvoir accéder aisément à la bande de scellage 5 sans que la coiffe 61 ne retombe, mais également d'obtenir un bras de levier important formé par la coiffe 61, reliée au capot 621 par le dispositif de liaison 64, qui est écartée de l'axe A22 de la charnière et dont la position est stable grâce à sa position pivotée en appui contre le capot 621. L'utilisateur peut ainsi ouvrir aisément le capot 621 par rapport à la partie de liaison 23 en se servant de la coiffe 61 comme bras de levier.

Selon un aspect particulier, l'angle de la position pivotée de la coiffe 61 est maintenu par engagement des ergots 6433 du dispositif de liaison 64 dans les logements 6216 de maintien formés à une extrémité de chacun des rails 6215 de la bague 6214.

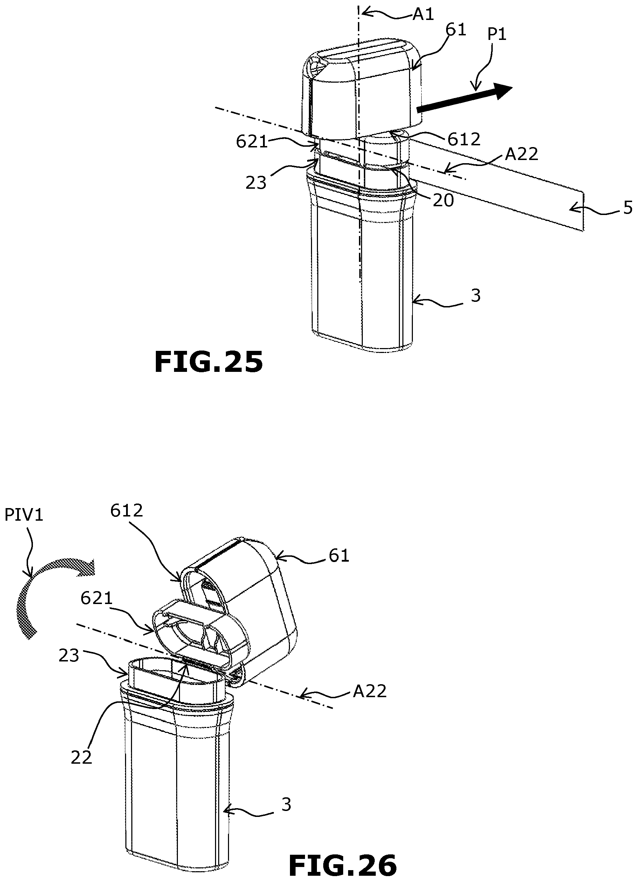

Comme illustré aux figures 25 et 26, l'utilisateur retire la bande de scellage 5, puis tire sur le capot 61 selon une direction transversale à l'axe A1 et à l'axe A22 de la charnière, par exemple selon la direction P1, pour faire pivoter l'ensemble de la coiffe 61 et du capot 621 afin d'ouvrir le capot 621 par rapport au corps creux 3,23. Le système de couplage 6432, 6214 entre le dispositif de liaison 64 et le capot 621 permet de transmettre au capot 621 l'effort appliqué à la coiffe 61 (en passant par le dispositif de liaison 64).

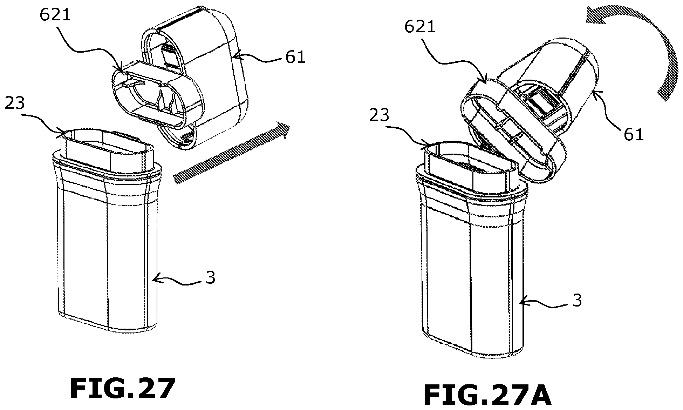

L'opérateur peut, comme illustré à la figure 27, détacher le capot 621, qui est pivoté en position ouverte, par rapport à la partie de liaison 23, en tirant sur le capot ou la coiffe jusqu'à la rupture de la charnière. En variante et comme illustré à la figure 27A, l'opérateur peut séparer le capot 621, qui est pivoté en position ouverte, par rapport à la partie de liaison 23, par un mouvement de torsion en tournant le capot 621 ou la coiffe 61 autour d'un axe transversal à la charnière jusqu'à la rupture de la charnière.

Grâce au dispositif de liaison entre la coiffe de protection et le capot, lorsque la coiffe de protection est déplacée en position d'ouverture (levée), en étant en outre de préférence pivotée par rapport au capot pour le mode de réalisation des figures 16 à 27A, la coiffe de protection reste liée au capot par le dispositif de liaison, tandis que le dispositif de liaison et la coiffe de protection forment un bras de levier associé au capot dont la longueur a augmenté par rapport à la position fermée de la coiffe, ce qui facilite l'ouverture du capot avec un effort réduit sur la coiffe.

Ce bras de levier permet à l'opérateur, une fois le système de fermeture retiré, défait ou cassé, lorsqu'il est présent, de faire pivoter aisément le capot autour de la charnière en appliquant un effort sur la partie d'extrémité de ce bras de levier formée par la coiffe de protection.

Après ouverture du capot, et de préférence détachement dudit capot, le contenu (emballage interne ou objet), peut être sorti de l'emballage.

L'éloignement de la coiffe par rapport au corps creux en position d'ouverture de la coiffe permet non seulement de former avec le dispositif de liaison ledit bras de levier relié au capot, mais également d'avoir, après ouverture à pivotement du capot, un accès aisé à l'intérieur du corps creux.

On peut en effet prévoir que, après ouverture, l'ensemble capot et coiffe soit pivoté autour de l'axe de la charnière par exemple à 180°, de sorte que les doigts de l'opérateur qui maintiennent le corps creux et l'emballage sont écartés de l'ouverture du corps creux.

Le détachement du capot, auquel la coiffe reste liée, permet de dégager la coiffe par rapport au corps creux restant qui loge le contenu, pour limiter le risque de contact inopiné avec ladite coiffe, pouvant générer un défaut d'asepsie, lors de la préhension du contenu (emballage interne ou objet) et/ou lors de sa sortie de l'emballage.

La zone d'accès à l'intérieur du contenant peut ainsi être correctement dégagée, ce qui permet de saisir le contenu sans risque de fautes d'asepsie.

Grâce au bras de levier formé, lorsque la coiffe de protection est en position d'ouverture, par l'ensemble de la coiffe de protection et du dispositif de liaison, l'effort à appliquer sur la coiffe de protection pour faire pivoter le capot est moins important que celui qu'il faudrait appliquer directement sur le capot en l'absence du dispositif de liaison de la coiffe de protection au capot.

Selon un aspect particulier, un dégagement complet de la coiffe (par exemple par pivotement à 180° autour de l'axe de la charnière ou par détachement par rapport au corps creux) permet d'écarter la coiffe de la zone d'accès correspondant à l'espace au-dessus de l'ouverture du corps creux (c'est-à-dire l'espace de sortie du contenu), ce qui permet de limiter le risque de faute d'asepsie lors de la saisie de l'objet, qui peut être dans un emballage interne. Une faute d'asepsie pourrait en effet résulter d'une proximité immédiate d'une partie résiduelle de la coiffe à proximité des doigts de l'opérateur stérile (par exemple l'infirmier (e) stérile ou le chirurgien).

### Variantes de dispositif de liaison

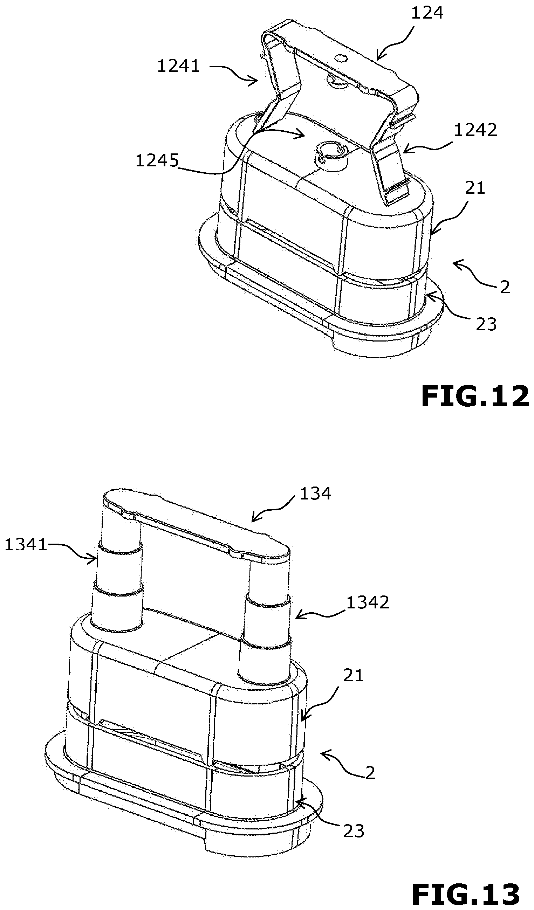

Selon une variante de réalisation illustrée à la figure 13, le dispositif de liaison est référencé 134 et comprend deux bras 1341, 1342 télescopiques.

La possibilité de faire varier la hauteur des bras selon l'axe A1 résulte alors de la possibilité de faire ressortir les éléments de chaque bras télescopique l'un par rapport à l'autre, alors que dans la version avec coude (figures 1 à 9), la variation de hauteur résulte de la possibilité de pliage/dépliage des bras au niveau des coudes.

Dans l'exemple illustré aux figures 1 à 9 présenté ci-avant, les traverses du dispositif de liaison sont des éléments du dispositif de liaison distincts de la coiffe de protection et du capot. En variante, on peut prévoir que ces traverses soient formées par des parties de la coiffe 1 de protection et/ou du capot 21. On peut en particulier prévoir que les bras du dispositif de liaison soient directement reliés à la coiffe 1 de protection et au capot 21.

Ainsi, dans l'exemple illustré à la figure 12, le dispositif de liaison (référencé 124) comprend une partie formant traverse supérieure et il peut être considéré que la traverse inférieure est formée par la partie supérieure du capot 21 à laquelle sont liées les bras (référencés 1241, 1242) du dispositif de liaison.

Dans l'exemple illustré plus particulièrement aux figures 2 et 5, le dispositif de liaison 4 et le capot 21 sont des pièces distinctes. Selon différents modes de réalisation, le dispositif de liaison 4 et le capot 21 peuvent être formés d'une seule pièce.

De manière similaire, dans l'exemple illustré à la figure 13, il peut être considéré que la traverse inférieure est formée par la partie supérieure du capot 21 à laquelle sont liées les bras (référencés 1341, 1342) du dispositif de liaison.

L'invention n'est pas limitée aux modes de réalisation illustrés dans les dessins. En conséquence, il doit être entendu que, lorsque les caractéristiques mentionnées dans les revendications annexées sont suivies par des signes de référence, ces signes sont inclus uniquement dans le but d'améliorer l'intelligibilité des revendications et ne sont nullement limitatifs de la portée des revendications.

De plus, le terme « comprenant » n'exclut pas d'autres éléments ou étapes. En outre, des caractéristiques ou étapes qui ont été décrites en référence à l'un des modes de réalisation exposés ci-dessus peuvent également être utilisées en combinaison avec d'autres caractéristiques ou étapes d'autres modes de réalisation exposés ci-dessus.

## Revendications

1. Emballage (6 ; 66) apte à contenir un objet, ledit emballage comprenant :
- un corps creux (3, 23) présentant une ouverture (230) de sortie dudit objet;
- un capot (21 ; 621) relié au corps creux (3, 23) par une charnière (22), permettant de fermer le corps creux (3, 23),
- une coiffe (1 ; 61) de protection, **caractérisé en ce que** ledit emballage (6 ; 66) comprend en outre un dispositif de liaison (4 ; 64) entre la coiffe (1 ; 61) de protection et le capot (21 ; 621),
la coiffe (1 ; 61) de protection étant déplaçable entre :
- une position de fermeture dans laquelle elle coiffe le capot (21 ; 621) et dans laquelle le dispositif de liaison (4 ; 64) présente une configuration réduite, et
- une position d'ouverture dans laquelle elle est écartée du corps creux (2, 23) et dans laquelle le dispositif de liaison (4 ; 64) est dans une configuration déployée,
la coiffe (1 ; 61) de protection formant avec le dispositif de liaison (4 ; 64),dans ladite position d'ouverture, un bras de levier couplé au capot (21 ; 621).

2. Emballage (6 ; 66) selon la revendication 1, dans lequel ledit emballage (6 ; 66) comprend un système de fermeture (5), retirable, défaisable ou cassable, permettant de maintenir le capot (21 ; 621) en position de fermeture du corps creux (3,23).

3. Emballage (6 ; 66) selon l'une quelconque des revendications 1 ou 2, dans lequel l'emballage (6 ; 66) comprend une bande de scellage (5) qui est appliquée sur les bords d'une fente (20) définie entre le capot (21 ; 621) et le corps creux (3, 23).

4. Emballage (6; 66) selon l'une quelconque des revendications 1 à 3, dans lequel, la charnière (22) présentant un axe (A22) et le corps creux (3, 23) présentant un axe (A1) de sortie dudit objet par ladite ouverture, le dispositif de liaison (4 ; 64) est rigide vis-à-vis d'un effort (P1) appliqué selon une direction transversale à l'axe (A1) de sortie du corps creux (3, 23) et transversale à l'axe (A22) de la charnière (22), pour permettre de faire pivoter le capot (21 ; 621).

5. Emballage (6 ; 66) selon l'une quelconque des revendications 1 à 4, dans lequel le dispositif de liaison (4 ; 64) comprend un système de maintien (45 ; 645) configuré pour, en position de fermeture de la coiffe (1 ; 61) de protection, maintenir le dispositif de liaison (4 ; 64) en configuration réduite, par exemple pliée ou contractée, tout en permettant une désactivation dudit système de maintien (45 ; 645) lors du passage de la coiffe (1 ; 61) de protection de ladite position de fermeture à ladite position d'ouverture qui entraine le passage du dispositif de liaison (4 ; 64) en configuration déployée.

6. Emballage (6 ; 66) selon l'une quelconque des revendications 1 à 5, dans lequel le dispositif de liaison (4 ; 64) se présente sous la forme d'un corps présentant un contour fermé déformable, de préférence plastiquement, par traction.

7. Emballage (6 ; 66) selon l'une quelconque des revendications 1 à 6, dans lequel le dispositif de liaison (4 ; 64) comprend deux bras (41, 42 ; 641, 642), chaque bras (41, 42 ; 641, 642) présentant un coude (411, 421 ; 6411, 6421).

8. Emballage (6 ; 66) selon la revendication 7, dans lequel chaque bras (41, 42 ; 641, 642) présente un amincissement de matière au niveau du coude (411, 421 ; 6411, 6421).

9. Emballage (6) selon l'une quelconque des revendications 7 à 8, dans lequel chaque bras (41, 42) présente un système d'accrochage (413, 415, 423, 425) configuré pour maintenir l'une contre l'autre les deux portions (412, 414, 422, 424) de bras (41, 42) qui s'étendent de part et d'autre du coude (411, 421) en position pliée du bras (42).

10. Emballage (66) selon l'une quelconque des revendications 7 à 9, dans lequel chaque bras (641, 642) présente un dispositif d'accrochage (6413, 6423) configuré pour, en coopération avec un dispositif d'accrochage (6217) correspondant ménagé sur le capot (621), maintenir ledit bras couplé au capot (621) en configuration réduite du dispositif de liaison (64).

11. Emballage (6 ; 66) selon l'une quelconque des revendications 7 à 10, prise en combinaison de la revendication 5, dans lequel, les deux bras (41, 42 ; 641, 642) étant reliés entre eux par une traverse inférieure (43 ; 643) et une traverse supérieure (44 ; 643), le système de maintien (45 ; 645) comprend un organe mâle (441 ; 6431) ménagé sur l'une (44 ; 643) des traverses et un organe femelle (431 ;6441) ménagé sur l'autre traverse (43 ; 644), lesdits organes mâle et femelle étant configurés pour coopérer par encliquetage l'un avec l'autre en position de fermeture de la coiffe (1 ; 61) de protection.

12. Emballage (6 ; 66) selon l'une quelconque des revendications 1 à 11, dans lequel le capot (21 ; 621) présente un logement d'insertion (214 ; 6214) configuré pour recevoir et maintenir une partie (43 ; 6432) du dispositif de liaison (4 ; 64) par rapport au capot (21 ; 621).

13. Emballage (66) selon l'une quelconque des revendications 1 à 12, dans lequel ledit emballage (66) comprend un système de couplage (6432 ; 6214) du dispositif de liaison (64) au capot (621), le système de couplage étant configuré de sorte que, à l'état déployé du dispositif de liaison (64), le dispositif de liaison (64) est pivotable par rapport au capot (621) de manière à pouvoir amener la bordure (612) de l'ouverture de la coiffe (61) en appui sur le capot (621).

14. Emballage (6 ; 66) selon l'une quelconque des revendications 1 à 13, dans lequel la coiffe (1 ; 61) de protection comprend deux parties (1A, 1B ; 61A, 61B) de coiffe qui sont assemblables l'une avec l'autre, pour permettre de monter une partie (44 ; 644) du dispositif de liaison (4 ; 64) dans un logement d'insertion (14 ; 614) de l'une (1A; 61A) des parties (1A, 1B ; 61A, 61B) de coiffe par une ouverture latérale de ladite partie (1A; 61A) de coiffe, et de refermer ladite ouverture latérale à l'aide de l'autre partie (1B ; 61B) de coiffe.

15. Emballage (6 ; 66) selon l'une quelconque des revendications 1 à 14, dans lequel est logé un emballage intérieur contenant un objet.

16. Emballage (6 ; 66) selon l'une quelconque des revendications 1 à 15, dans lequel est logé un dispositif de préhension auquel est couplé ledit objet.

17. Procédé de déconditionnement d'un objet logé dans un emballage (6 ; 66) selon l'une quelconque des revendications 1 à 16, dans lequel ledit procédé comprend les étapes suivantes :
- déplacement de la coiffe (1 ; 61) de protection en position d'ouverture, la coiffe (1 ; 61) de protection restant liée au capot (21 ; 621) par le dispositif de liaison (4 ; 64) ;
- application d'un effort (P1) sur la coiffe (1 ; 61) de protection pour faire pivoter (PIV1) le capot (21 ; 621) autour de l'axe (A22) de la charnière (22).

18. Procédé selon la revendication 17, dans lequel l'emballage (66) est conforme à la revendication 13 et dans lequel ledit procédé comprend en outre, entre lesdites étapes de déplacement de la coiffe (61) de protection et d'application d'un effort sur la coiffe (61) de protection pour faire pivoter le capot, une étape de pivotement (PIV61) de la coiffe (61) de protection pour amener la bordure (612) de l'ouverture de la coiffe (61) de protection en appui sur le capot (621).

19. Procédé selon la revendication 17 ou 18, dans lequel le procédé comprend en outre après l'étape de déplacement de la coiffe (1 ; 61) de protection, une étape de retrait d'une bande de scellage (5) appliquée sur les bords d'une fente (20) définie entre le capot (21 ;621) et le premier corps creux (3, 23).

20. Procédé selon l'une quelconque des revendications 17 à 19, dans lequel le procédé comprend en outre une étape de séparation du capot (21 ; 621) par rapport au corps creux (3, 23) par application d'un effort de traction ou torsion sur le capot (21 ; 621) et/ou la coiffe (1 ; 61) de protection.

## Patentansprüche

1. Verpackung (6; 66), die imstande ist, einen Gegenstand zu enthalten, wobei die Verpackung umfasst:
- einen Hohlkörper (3, 23), der eine Ausgangsöffnung (230) des Gegenstands aufweist;
- einen Deckel (21; 621), der mit dem Hohlkörper (3, 23) durch ein Scharnier (22) verbunden ist, so dass der Hohlkörper (3, 23) verschlossen werden kann,
- eine Schutzkappe (1; 61),
**dadurch gekennzeichnet, dass** die Verpackung (6; 66) ferner eine Verbindungsvorrichtung (4; 64) zwischen der Schutzkappe (1; 61) und dem Deckel (21; 621) umfasst,
wobei die Schutzkappe (1; 61) verlagerbar ist zwischen:
- einer Verschlussposition, in welcher sie den Deckel (21; 621) bedeckt und in welcher die Verbindungsvorrichtung (4; 64) eine reduzierte Konfiguration aufweist, und
- eine Öffnungsposition, in welcher sie vom Hohlkörper (2, 23) beabstandet ist und in welcher die Verbindungsvorrichtung (4; 64) in einer entfalteten Konfiguration ist,
wobei die Schutzkappe (1; 61) mit der Verbindungsvorrichtung (4; 64) in der Öffnungsposition einen an den Deckel (21; 621) gekoppelten Hebelarm bildet.

2. Verpackung (6; 66) nach Anspruch 1, wobei die Verpackung (6; 66) ein herausziehbares, entfernbares oder zerstörbares Verschlusssystem (5) umfasst, das erlaubt, den Deckel (21; 621) in Verschlussposition des Hohlkörpers (3, 23) zu halten.

3. Verpackung (6; 66) nach einem der Ansprüche 1 oder 2, wobei die Verpackung (6; 66) ein Siegelband (5) umfasst, das auf den Rändern eines Schlitzes (20) angebracht ist, der zwischen dem Deckel (21; 621) und dem Hohlkörper (3, 23) definiert ist.

4. Verpackung (6; 66) nach einem der Ansprüche 1 bis 3, wobei, wobei das Scharnier (22) eine Achse (A22) aufweist und der Hohlkörper (3, 23) eine Ausgangsachse (A1) des Gegenstands durch die Öffnung aufweist, die Verbindungsvorrichtung (4; 64) gegenüber einer Kraft (P1), die in einer Richtung angewendet wird, die zur Ausgangsachse (A1) des Hohlkörpers (3, 23) transversal ist und zur Achse (A22) des Scharniers (22) transversal ist, starr ist, um zu gestatten, den Deckel (21; 621) zu schwenken.

5. Verpackung (6; 66) nach einem der Ansprüche 1 bis 4, wobei die Verbindungsvorrichtung (4; 64) ein Haltesystem (45; 645) umfasst, das dazu ausgelegt ist, in Verschlussposition der Schutzkappe (1; 61) die Verbindungsvorrichtung (4; 64) in reduzierte Konfiguration zu halten, beispielsweise gefaltet oder eingezogen, bei Gestattung einer Deaktivierung des Haltesystems (45; 645) beim Wechsel der Schutzkappe (1; 61) aus der Verschlussposition in die Öffnungsposition, der den Wechsel der Verbindungsvorrichtung (4; 64) in entfaltete Konfiguration bewirkt.

6. Verpackung (6; 66) nach einem der Ansprüche 1 bis 5, wobei die Verbindungsvorrichtung (4; 64) in Form eines Körpers vorliegt, der eine durch Zug vorzugsweise plastisch verformbare geschlossene Kontur aufweist.

7. Verpackung (6; 66) nach einem der Ansprüche 1 bis 6, wobei die Verbindungsvorrichtung (4; 64) zwei Arme (41, 42; 641, 642) umfasst, wobei jeder Arm (41, 42; 641, 642) einen Knick (411, 421; 6411, 6421) aufweist.

8. Verpackung (6; 66) nach Anspruch 7, wobei jeder Arm (41, 42; 641, 642) eine Materialverschlankung im Bereich des Knicks (411, 421; 6411, 6421) aufweist.

9. Verpackung (6) nach einem der Ansprüche 7 bis 8, wobei jeder Arm (41, 42) ein Verankerungssystem (413, 415, 423, 425) aufweist, das dazu ausgelegt ist, die zwei Abschnitte (412, 414, 422, 424) der Arme (41, 42), die sich beiderseits des Knicks (411, 421) erstrecken, in gefalteter Position des Arms (42) gegeneinander zu halten.

10. Verpackung (66) nach einem der Ansprüche 7 bis 9, wobei jeder Arm (641, 642) eine Verankerungsvorrichtung (6413, 6423) aufweist, die dazu ausgelegt ist, im Zusammenwirken mit einer entsprechenden Verankerungsvorrichtung (6217), die auf dem Deckel (621) eingerichtet ist, in reduzierter Konfiguration der Verbindungsvorrichtung (64) den Arm am Deckel (621) gekoppelt zu halten.

11. Verpackung (6; 66) nach einem der Ansprüche 7 bis 10, herangezogen in Kombination mit Anspruch 5, wobei, wobei die zwei Arme (41, 42; 641, 642) durch eine untere Traverse (43; 643) und eine obere Traverse (44; 643) miteinander verbunden sind, das Haltesystem (45; 645) ein männliches Element (441; 6431) umfasst, das auf der einen (44; 643) der Traversen eingerichtet ist, und ein weibliches Element (431; 6441), das auf der anderen Traverse (43; 644) eingerichtet ist, wobei das männliche und weibliche Element dazu ausgelegt sind, durch Rasten miteinander in Verschlussposition der Schutzkappe (1; 61) zusammenzuwirken.

12. Verpackung (6; 66) nach einem der Ansprüche 1 bis 11, wobei der Deckel (21; 621) eine Aufnahme (214; 6214) aufweist, die dazu ausgelegt ist, einen Teil (43; 6432) der Verbindungsvorrichtung (4; 64) im Verhältnis zum Deckel (21; 621) zu empfangen und zu halten.

13. Verpackung (66) nach einem der Ansprüche 1 bis 12, wobei die Verpackung (66) ein Kopplungssystem (6432; 6214) der Verbindungsvorrichtung (64) an den Deckel (621) umfasst, wobei das Kopplungssystem derart ausgelegt ist, dass die Verbindungsvorrichtung (64) im entfalteten Zustand der Verbindungsvorrichtung (64) im Verhältnis zum Deckel (621) derart schwenkbar ist, dass der Rand (612) der Öffnung der Schutzkappe (61) in Abstützung auf den Deckel (621) führbar ist.

14. Verpackung (6; 66) nach einem der Ansprüche 1 bis 13, wobei die Schutzkappe (1; 61) zwei Schutzkappenteile (1A, 1B; 61A, 61B) umfasst, die miteinander verbindbar sind, um die Anbringung eines Teils (44; 644) der Verbindungsvorrichtung (4; 64) in einer Aufnahme (14; 614) von einem (1A; 61A) der Schutzkappenteile (1A, 1B; 61A, 61B) durch eine Seitenöffnung des Schutzkappenteils (1A; 61A) zu erlauben und die Seitenöffnung mit Hilfe des anderen Schutzkappenteils (1B; 61B) zu verschließen.

15. Verpackung (6; 66) nach einem der Ansprüche 1 bis 14, in der eine Innenverpackung untergebracht ist, die einen Gegenstand enthält.

16. Verpackung (6; 66) nach einem der Ansprüche 1 bis 15, in der eine Greifvorrichtung untergebracht ist, an die der Gegenstand gekoppelt ist.

17. Verfahren zum Entpacken eines in einer Verpackung (6; 66) nach einem der Ansprüche 1 bis 16 untergebrachten Gegenstands, wobei das Verfahren die folgenden Schritte umfasst:
- Verlagern der Schutzkappe (1; 61) in Öffnungsposition, wobei die Schutzkappe (1; 61) durch die Verbindungsvorrichtung (4; 64) mit dem Deckel (21; 621) verbunden bleibt;
- Anwenden einer Kraft (P1) auf die Schutzkappe (1; 61), um den Deckel (21; 621) um die Achse (A22) des Scharniers (22) zu schwenken (PIV1).

18. Verfahren nach Anspruch 17, wobei die Verpackung (66) nach Anspruch 13 ist und wobei das Verfahren ferner zwischen den Schritten des Verlagerns der Schutzkappe (61) und des Anwendens einer Kraft auf die Schutzkappe (61), um den Deckel zu schwenken, einen Schritt des Schwenkens (PIV61) der Schutzkappe (61) umfasst, um den Rand (612) von der Öffnung der Schutzkappe (61) in Abstützung auf den Deckel (621) zu führen.

19. Verfahren nach Anspruch 17 oder 18, wobei das Verfahren ferner nach dem Schritt des Verlagerns der Schutzkappe (1; 61) einen Schritt des Entfernens eines Siegelbands (5) umfasst, das auf den Rändern eines Schlitzes (20) angebracht ist, der zwischen dem Deckel (21; 621) und dem ersten Hohlkörper (3, 23) definiert ist.

20. Verfahren nach einem der Ansprüche 17 bis 19, wobei das Verfahren ferner einen Schritt des Entfernens des Deckels (21; 621) im Verhältnis zum Hohlkörper (3, 23) durch Anwenden einer Zug- oder Torsionskraft auf den Deckel (21; 621) und/oder die Schutzkappe (1; 61) umfasst.

## Claims

1. A package (6; 66) able to contain an object, said package comprising:
- a hollow body (3, 23) having an opening (230) through which said object exits;
- a lid (21; 621) joined to the hollow body (3, 23) by a hinge (22), enabling the hollow body (3, 23) to close;
- a protective cover (1; 61);
**characterized in that**
said package (6; 66) further comprises a coupling device (4; 64) between the protective cover (1; 61) and the lid (21; 621),
the protective cover (1; 61) being movable between:
- a closed position in which it covers the lid (21; 621) and in which the coupling device (4; 64) assumes a retracted configuration; and
- an open position in which it is spaced apart from the hollow body (2, 23) and in which the coupling device (4; 64) assumes a deployed configuration,
wherein the protective cover (1; 61) forms, with the coupling device (4; 64), in said open position, a lever arm coupled to the lid (21; 621).

2. The package (6; 66) according to claim 1, wherein said package (6; 66) comprises a closure system (5) that can be removed, undone or broken, thereby enabling the lid (21; 621) to be kept in the position closing the hollow body (3, 23).

3. The package (6; 66) according to any one of claims 1 or 2, wherein said package (6; 66) comprises a sealing strip (5) that is applied on the edges of a slot (20) defined between the lid (21; 621) and the hollow body (3, 23).

4. The package (6; 66) according to any one of claims 1 to 3, wherein the hinge (22) having an axis (A22) and the hollow body (3, 23) having an axis (A1) by which said object exits through said opening, the coupling device (4; 64) is rigid with respect to a force (P1) applied in a direction that is transverse to the axis (A1) by which the hollow body (3, 23) exits and transverse to the axis (A22) of the hinge (22), to enable the lid (21; 621) to pivot.

5. The package (6; 66) according to any one of claims 1 to 4, wherein the coupling device (4; 64) comprises a fastening system (45; 645) designed to keep the coupling device (4; 64) in the retracted configuration, for example folded or contracted, when the protective cover (1; 61) is in the closed position, while enabling said fastening system (45; 645) to be disabled when the protective cover (1; 61) is moved from said closed position to said open position, which causes the coupling device (4; 64) to assume the deployed configuration.

6. The package (6; 66) according to any one of claims 1 to 5, wherein the coupling device (4; 64) is in the form of a body with a closed contour that can be deformed, preferably plastically, by pulling.

7. The package (6; 66) according to any one of claims 1 to 6, wherein the coupling device (4; 64) comprises two arms (41, 42; 641, 642), with each arm (41, 42; 641, 642) having a bend (411, 421; 6411, 6421).

8. The package (6; 66) according to claim 7, wherein each arm (41, 42; 641, 642) has thinner material at the bend (411, 421; 6411, 6421).

9. The package (6) according to any one of claims 7 to 8, wherein each arm (41, 42) has a hooking system (413, 415, 423, 425) configured to hold the two portions (412, 414, 422, 424) of the arms (41, 42) that extend on either side of the bend (411, 421) together when the arm (42) is in the folded position.

10. The package (66) according to any one of claims 7 to 9, wherein each arm (641, 642) has a hooking device (6413, 6423) configured, in cooperation with a corresponding hooking device (6217) provided on the lid (621), to keep said arm coupled to the lid (621) in a retracted configuration of the coupling device (64).

11. The package (6; 66) according to any one of claims 7 to 10, taken in combination with claim 5, wherein, as the two arms (41, 42; 641, 642) are connected by a lower crossbar (43; 643) and an upper crossbar (44; 643), the fastening system (45; 645) has a male part (441; 6431) arranged on one (44; 643) of the crossbars and a female part (431; 6441) arranged on other crossbar (43; 644), said male and female parts being configured to work together by clicking into one another when the protective cover (1; 61) is in the closed position.

12. The package (6; 66) according to any one of claims 1 to 11, wherein the lid (21; 621) has an insertion housing (214; 6214) configured to receive and hold a part (43; 6432) of the coupling device (4; 64) with regard to the lid (21; 621).

13. The package (66) according to any one of claims 1 to 12, wherein said package (66) comprises a coupling system (6432; 6214) of the coupling device (64) for coupling to the lid (621), the coupling system being configured so that, in the deployed state of the coupling device (64), the coupling device (64) can be pivoted relative to the lid (621) so that the edge (612) of the opening of the cover (61) can rest on the lid (621).

14. The package (6; 66) according to any one of claims 1 to 13, wherein the protective cover (1; 61) comprises two cover parts (1A, 1B; 61A, 61B) that are able to be assembled with one another to enable a part (44; 644) of the coupling device (4; 64) to be mounted to the insertion housing (14; 614) of one (1A; 61A) of the cover parts (1A, 1B; 61A, 61B) by a lateral opening in said cover part (1A; 61A), and to close said lateral opening by means of the other cover part (1B; 61B).

15. The package (6; 66) according to any one of claims 1 to 14, wherein an inner package containing an object is accommodated.

16. The package (6; 66) according to any one of claims 1 to 15, wherein a gripping device coupled to said object is accommodated.

17. A method for unpacking an object accommodated in a package (6; 66) according to any one of claims 1 to 16, wherein said method comprises the following steps:
- moving the protective cover (1; 61) to the open position, the protective cover (1; 61) remaining joined to the lid (21; 621) by the coupling device (4; 64); and
- applying a force (P1) on the protective cover (1; 61) to cause the lid (21; 621) to pivot (PIV1) about the axis (A22) of the hinge (22).

18. The method according to claim 17, wherein the package (66) is according to claim 13 and wherein said method further comprises, between said steps of moving the protective cover (61) and applying a force on the protective cover (61) for pivoting the lid, a step of pivoting (PIV61) the protective cover (61) to bring the edge (612) of the opening of the protective cover (61) to rest on the lid (621).

19. The method according to claim 17 or 18, wherein the method further comprises, after the step of moving the protective cover (1; 61), a step of removing a sealing strip (5) applied to the edges of a slot (20) defined between the lid (21; 621) and the first hollow body (3, 23).

20. The method according to any one of claims 17 to 19, wherein the method further comprises a step of separating the lid (21; 621) from the hollow body (3, 23) by applying a pulling or twisting force on the lid (21; 621) and/or on the protective cover (1; 61).
